# EUROPEAN PATENT APPLICATION

(11) **EP 1 398 031 A1**
(43) Date of publication of application: **17.03.2004**
(21) Application number: 02020087.9
(22) Date of filing: 06.09.2002
(51) Int. Cl.: A61K 31/47, A61K 31/7088, A61P 35/00

(54) **Use of 6-amino-quinoline-5,8-quinones and nucleic acids associated with senescence for the treatment of tumors**

(71) Applicant: Max-Planck-Gesellschaft zur Förderung der Wissenschaften e.V., 80539 München (DE)
(72) Inventor: Hermeking, Heiko, Dr., 82152 Martinsried (DE); Lodygin, Dimitri Max-Planck-Inst. für Biochemie, 85152 Martinsried/Planegg (DE)
(74) Representative: Weiss, Wolfgang, Dipl.-Chem. Dr.

(57) **Abstract**

The present invention relates to the use of at least one 6-amino-quinoline-5,8-quinone for preparing a pharmaceutical composition for the treatment of tumors. A further subject matter of the present invention is a nucleic acid associated with senescence and its use for the treatment of tumors.

## Description

The present invention relates to the use of at least one 6-amino-quinoline-5,8-quinone for preparing a pharmaceutical composition for the treatment of tumors. A further subject matter of the present invention are nucleic acids associated with senescence and their use for the treatment of tumors.

Several cellular mechanisms have evolved to prevent proliferation of cells, which have acquired mutations with carcinogenic potential. One mechanism is represented by apoptosis: cells with deregulated expression of oncogenes are removed through this route (Green and Evan, 2002). Another cellular response, that gained more attention recently, is senescence: a state of terminal arrest in which cells are metabolically active for extended periods, but are unable to re-enter the cell cycle after mitogenic stimulation (Hayflick and Moorehead, 1961; Shay and Wright, 2000). Several tumor-suppressor genes (e.g. *p53* and *p16*^{*INK4A*}*)* are involved in the induction and maintenance of senescence, strongly suggesting that senescence is a tumor suppressive mechanism (Serrano and Blasco, 2001; Campisi, 2001). Supporting this notion on the organismal level, mice, which have been engineered to display elevated p53 activity, show a decreased rate of tumor-formation and premature aging (Tyner et *al.,* 2002).

Replicative senescence results from progressive shortening of chromosomes, which occurs during every cell division. After 50-70 cell divisions, the loss of telomeric DNA repeats results in unprotected chromosome ends, which generate a DNA-damage signal resulting in activation of the tumor suppressor gene product p53. Therefore, several downstream targets of the p53 transcription factor are induced during replicative senescence, e.g. *p21*^{*WAF1*}^{/}^{*SDI*}^{/}^{*CIP1*} and *14-3-3sigma* (Noda et *al.,* 1994; Dellambra *et al.,* 2000). Consistent with an essential function of these p53 target-genes during senescence, deletion of *p21* through homologous recombination is sufficient to bypass replicative senescence in human diploid fibroblasts (Brown *et al*., 1997). Similarly, inactivation of *14-3-3sigma* leads to immortalization of primary human keratinocytes (Dellambra *et al.,* 2000).

In contrast to telomer-dependent replicative senescence, cellular senescence, also referred to as premature senescence, is a response to diverse stimuli (e.g. oncogenic signalling, sub-optimal tissue culture conditions), which generally involves induction of the tumor suppressor gene *p16*/*INK4.* Cellular senescence accompanied by induction of *p16* is also observed after serial passaging of epithelial cells under standard tissue culture conditions. Interestingly, the *p16* gene is not induced in epithelial cells, when these are cultivated on feeder-layers: under these conditions epithelial cells have a greatly elevated replicative potential and terminally arrest due to shortened telomeres, i.e. replicative senescence (Ramirez *et* al., 2001 ) . In contrast to epithelial cells, primary human fibroblasts undergo replicative senescence under standard tissue culture conditions (reviewed in Christofalo *et al.,* 1998). These examples point to the existence of cell-type dependent signalling pathways, which activate the senescence program.

DNA-damaging agents commonly used for cancer therapy induce a senescence-like state in normal and cancer cells (Di Leonardo *et al.,* 1994; Chang *et al.,* 1999). However, since these substances also induce DNA-damage in normal proliferating cells, numerous unwanted side effects are observed during chemotherapy (Bast *et al.,* 2000). Furthermore, these agents generate mutations in pre-cancerous cells, which may result in secondary cancer. In the studies underlying the present invention, we aimed to identify substances, which activate the cellular senescence without inducing DNA-damage. We hypothesized that pharmacological inhibition of signalling pathways, which are specifically down-regulated during replicative senescence, may result in the reactivation of the senescence program in tumor cells. The ideal drug-target for this strategy would be an enzyme encoded by a gene that is repressed during senescence. Inhibition of such an enzyme by a small, membrane permeable drug molecule in early passage or tumor cells should theoretically be sufficient to induce cellular senescence. In order to detect genes and pathways repressed during replicative senescence, the gene expression pattern of senescent human fibroblasts was compared to the expression signature of confluent, early passage cells. Using microarray analysis, we have discovered within the framework of the present invention down-regulation of several components of the cGMP- signalling pathway during replicative senescence of primary human diploid fibroblasts (HDF). Therefore, the effect of pharmacological inhibition of cGMP-synthesis was analyzed in HDF. 6-Anilino-quinoline-5,8-quinone (LY83583 or LY), an inhibitor of guanylate cyclase, unexpectedly induced cellular senescence. 114 genes differentially expressed after treatment with LY were regulated similarly in HDF undergoing replicative senescence, indicating that these genes may constitute components of a transcriptional program, which mediates the senescent phenotype. Among the LY-induced genes was the cdk-inhibitor *p21*^{*WAF1*}^{/}^{*SDI*}^{/}^{*CIP1*}. In colorectal cancer cells, transcription of *p21* was induced by LY in a *p53*-independent manner. Furthermore, *p21* but not p53, was required for cell-cycle inhibition by LY. Inactivation of the retinoblastoma tumor suppressor protein, an effector of p21-mediated cell cycle inhibition, converted LY-induced growth arrest to apoptosis. These results suggest that LY, or derivatives thereof, may be useful tumor therapeutics.

Therefore, a first subject matter of the invention relates to the use of at least one 6-amino-quinoline-5,8-quinone for preparing a pharmaceutical composition for the treatment of tumors.

The pharmaceutical composition of the invention constitutes a new class of non-genotoxic anti-tumor drugs which are capable of inducing cellular senescence and/or apoptosis in tumor cells. An obvious advantage of said pharmaceutical composition is its ability to interfere with cellular proliferation without inducing DNA damage. Commonly used chemotherapeutics like, e.g. adriamycin, cause DNA damage and thereby selectively induce apoptosis in tumor cells which lack cell cycle checkpoints. However, DNA-damaging substances cause major side effects on proliferating tissues and may induce further mutations in pre-cancerous and healthy cells which lead to secondary cancer.

The pharmaceutical composition of the invention can contain a single 6-amino-quinoline-5,8-quinone or a combination of several 6-amino-quinoline-5,8-quinones. Furthermore, the pharmaceutical composition can contain conventionally used pharmaceutical additives well-known to the skilled artisan, if desired, such as, for example, physiologically acceptable carrier substances, diluents and adjuvants.

In a preferred embodiment of the invention the 6-amino-quinoline-5,8-quinone is 6-anilino-quinoline-5,8-quinone (LY83583 or LY).

The pharmaceutical composition of the invention can be topically, parenterally, intravenously, intramuscularly, subcutaneously or transdermally applicable and can be produced according to conventional methods well-known in the art. Preferably, the pharmaceutical composition of the invention is produced in the form of tablets or as an intravenous injection or infusion.

The pharmaceutical composition according to the invention is used for the treatment of tumors. The term "tumor", as used herein, comprises any local increase in tissue volume, i.e., in the broadest sense, any localized swelling due to oedema, acute and chronic inflammation, aneurysmal dilatation and organ swelling caused by inflammation as well as, in the narrowest sense, formation of new tissue (e.g. tumor, blastoma, neoplasia) in the form of spontaneous, differently disinhibited, autonomous and irreversible excess growth of endogenous tissue which normally is associated with loss of specific cell and tissue functions in various degrees. Examples of tumor diseases which can be treated by means of the pharmaceutical composition according to the invention comprise intestinal cancer, brain tumor, eye tumor, pancreatic tumor, urinary bladder carcinoma, lung cancer, breast cancer, ovarian tumor, uterine carcinoma, bone tumor, gall bladder and bile duct carcinoma, head-neck tumor, urethral cancer, skin cancer, testicular cancer, renal tumor, germinal tumor, liver carcinoma, leucemia, gastric carcinoma, malignant lymphoma, adrenal carcinoma, neuroma, neuroblastoma, malignant melanoma, oesophageal cancer, prostate carcinoma, soft-tissue tumor, thymoma and carcinoma in the case of unknown primary cancer.

The expression "treatment of tumors", as used herein, comprises at least one of the following features: alleviation of the symptoms associated with the tumor disease, reduction of the extent of the tumor disease (e.g. reduction of tumor growth), stabilization of the state of the tumor disease (e.g. inhibition of tumor growth), inhibition of proliferation of the tumor disease (e.g. metastasis), deceleration or retardation of the progress of the tumor disease, improvement of the state of the tumor disease (e.g. reduction of the tumor) or prevention of the occurrence or reoccurrence of a tumor disease.

Preferably, the pharmaceutical composition of the invention is administered to a patient with a tumor disease, with a presumed tumor disease or with a high tumor risk in an amount sufficient to achieve treatment of the tumor or prevention of a tumor. Particularly preferred, the pharmaceutical composition is administered to a patient suffering from a tumor disease in a therapeutically effective amount. The amount of the pharmaceutical composition to be administered thereby depends on several factors, e.g. on the 6-amino-quinoline-5,8-quinone chosen, the type of administration (tablet, injection, infusion, etc.), the kind and extent of the tumor disease and the age, weight and general condition of the patient and can be easily determined by one skilled in the field of tumor diseases, considering the above-mentioned factors. Preferably, however, the complexes of the invention are administered in the range of from 1 µg/kg body weight of the patient to 5 mg/kg body weight of the patient, preferably from 1 µg/kg body weight of the patient to 0.5 mg/kg body weight of the patient and, especially preferred, from 10 µg/kg body weight of the patient to 0.1 mg/kg body weight of the patient.

The pharmaceutical composition of the invention is administered topically, parenterally, intravenously, intramuscularly, subcutaneously or transdermally. Preferably, the pharmaceutical composition is administered in the form of tablets or as intravenous injection or infusion. In isolated cases the pharmaceutical composition can be injected specifically in body cavities or via a catheter in the blood vessels of the tumor region or the organ where the tumor is located.

Preferably, the pharmaceutical composition of the invention leads to inhibition of tumor cell proliferation by 6-amino-quinoline-5,8-quinone-induced activation of cellular senescence, cell cycle arrest and/or apoptosis. By means of the studies underlying the present invention it could be shown that 6-anilino-quinoline-5,8-quinone inhibits tumor cell proliferation by induction of p21 in a p53-independent manner. Additional inactivation of the retinoplasma tumor suppressor protein (RB protein), occuring e.g. in many tumor cells which have lost RB protein function either through mutation in RB protein or through expression of viral proteins, converted the 6-anilino-quinoline-5,8-quinone-induced growth arrest to apoptosis.

Particularly preferred, the composition of the invention leads to inhibition or reduction of tumor growth, reduction of tumor mass or inhibition or reduction of metastatic spread due to 6-amino-quinoline-5,8-quinone-induced activation of cellular senescence.

Furthermore, a second subject matter of the present invention relates to a nucleic acid associated with senescence characterized in that it encodes one of the proteins shown in Table 2 or functionally active variants or fragments thereof.

The studies underlying the present application could observe 114 differently expressed genes during replicative senescence and during 6-anilino-quinoline-5,8-quinone-induced cellular senescence (Table 2). Among these were genes which had previously been identified as differently regulated during senescence: PAI-1, metallix metalloproteinase 10, fibrillin, cdc25b, cyclin D1, fibromodulin and osf-2, and which therefore do not represent nucleic acids according to the invention. However, most of the genes identified represent new additions to the growing number of genes identified as components of the senescence program.

The nucleic acid according to the invention is a senescence-associated nucleic acid, i.e. a nucleic acid which is associated with the occurrence of replicative or cellular senescent processes in a cell, especially in a mammalian cell, and which encodes one of the proteins or functionally active variants or fragments thereof, as shown in Table 2. The nucleic acid can be from any organism, with eukaryotic organisms such as nematodes, e.g. C.elegans, arthropodes such as Drosophila, and vertebrates, e.g. mammals, being preferred. Particularly preferred, it is a mammalian sequence, e.g. human, whereby said sequence can be modified, if desired, by known molecular biological techniques such as site-specific mutagenesis, PCR, restriction cleavage and ligation. The nucleic acid can be double-stranded or single-stranded, e.g. in the form of DNA or RNA. Preferably, the nucleic acid is a senescence-inducing nucleic acid, i.e. a nucleic acid causing and/or enhancing senescence. Particularly preferred, the nucleic acid of the invention is a dominantly inducing nucleic acid which is capable of inducing cellular senescence upon expression in a cell and of producing the characteristic features of senescence, i.e. cell cycle arrest, in which cells are metabolically active for extended periods but are unable to re-enter the cell cycle after mitogenic stimulation.

Preferably, the nucleic acid of the invention is operatively linked with an expression control sequence so that it can be transcribed and translated, if desired, in a suitable host cell. Particularly preferred, the expression control sequence is a heterologous expression control sequence. Expression control sequences usually comprise a promoter and regulatory sequences, if desired, such as operators or enhancers. Furthermore, translation initation sequences may be present. Suitable expression control sequences are well-known to the skilled artisan.

Another subject matter of the present invention is a recombinant vector containing a nucleic acid according to the invention which is operatively linked with an expression control sequence. The recombinant vector can also contain usual elements such as an origin of replication and a selection marker. Examples of suitable recombinant vectors, e.g. plasmids, cosmids, phages, viruses, etc. are known to the skilled artisan.

Still another subject matter of the invention is a recombinant cell which is transformed or transfected with a nucleic acid or a vector according to the invention. Transformation and transfection, respectively, can be effected according to known methods, e.g. by calcium phosphate coprecipitation, lipofection, electroporation, particle bombardment or viral infection. The cell of the invention can contain the recombinant nucleic acid in extrachromosomal or chromosomally integrated form.

A still further subject matter of the present invention is a senescence-associated polypeptide encoded by a nucleic acid of the invention. A senescence-associated polypeptide can be obtained by expressing the senescence-associated nucleic acid of the invention, by chemical synthesis or by combining these two methods.

Further, one subject matter of the invention is a pharmaceutical composition containing a nucleic acid according to the invention, a vector according to the invention or a polypeptide according to the invention, optionally together with usual pharmaceutical carrier substances and auxiliary substances. The pharmaceutical composition, in particular, can be used for the treatment and prevention of diseases characterized by hyperproliferation such as, for example, tumor diseases, autoimmune diseases and viral infections. Preferably, the pharmaceutical composition is used for the treatment of tumor diseases. In this context reference is made to pages 4 to 7 of the present application which also apply here, too.

Another subject matter of the present invention, finally, is the use of a nucleic acid according to the invention, a vector according to the invention or a protein according to the invention for preparing a pharmaceutical composition for the treatment of diseases associated with hyperproliferation. Preferably, the pharmaceutical composition serves for the treatment of tumors. In this context, reference is also made to pages 4 to 7 of the present application which apply here, too.

The present invention is further illustrated by the following Figures, Tables and Examples.

### Tables

Table 1 shows a microarray analysis of senescent human diploid fibroblasts. Gene expression patterns of early passage confluent HDF were compared to senescent HDF using microarrays as described in the Examples. **A.** Detection of differential regulation of genes previously known to be associated with senescence. **B.** Repression of genes encoding components of the cGMP signalling pathway. Confirmation of expression data by quantitative real-time PCR is indicated in the column labeled "qPCR" including standard deviations in brackets.

Table 2 shows 114 mRNAs co-regulated in replicative senescence and LY-treated cells detected by microarray analysis. Genes, which showed a similar differential regulation (> 1.7 fold induction or repression) in the two microarray analyses, are shown after classification according to their function. For each gene, fold differential expression during replicative senescence (repl. sen.) or after LY treatment is shown.

### Figures

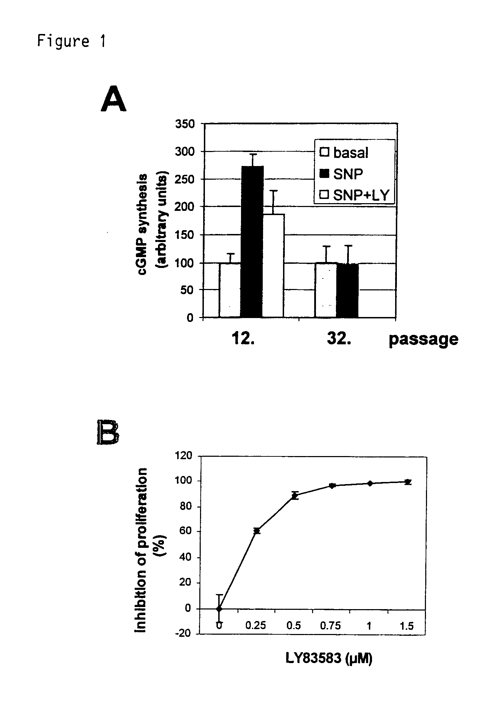

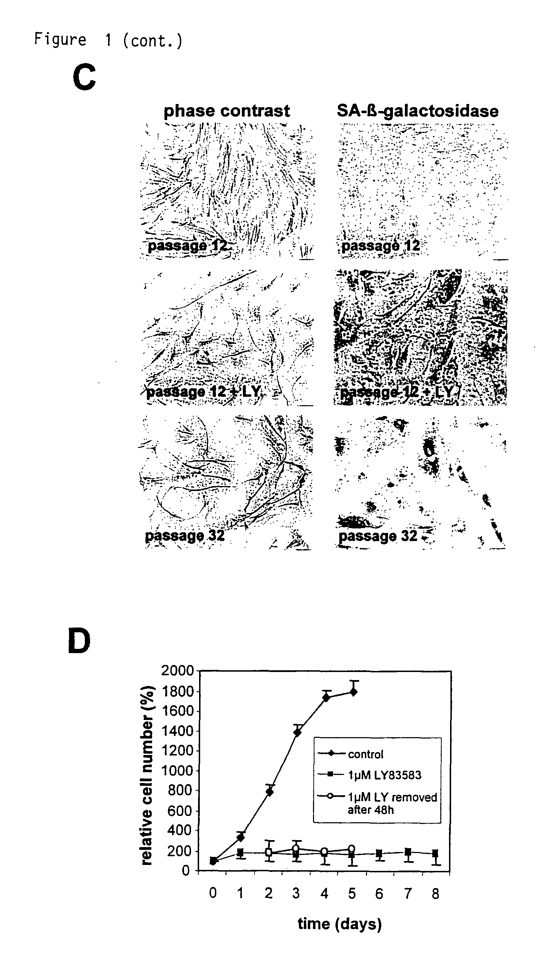

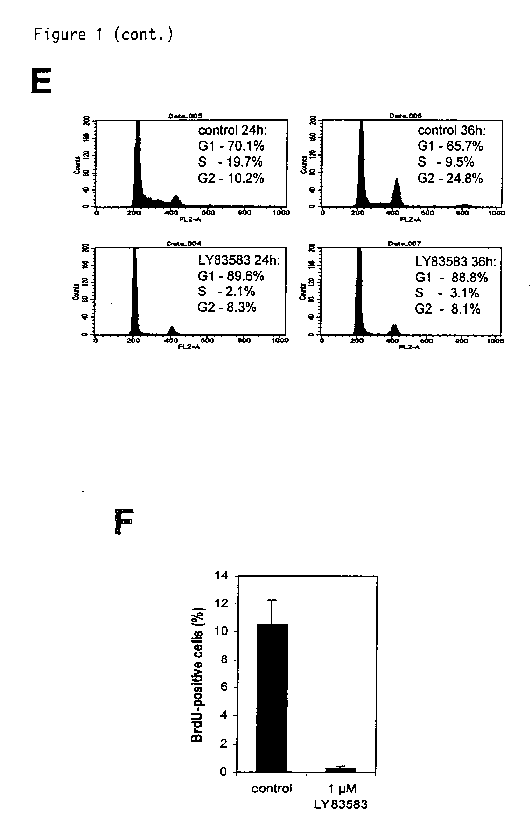

Fig. 1 shows the modulation of the cGMP-pathway in HDF effected by LY. **A:** Response of early passage and senescent HDF to activators of cGMP-synthesis. Cells were treated with 100 µM SNP (sodium nitroprusside) for 2 hours in the absence or presence of LY. cGMP levels were determined as described in the Examples. Treatments were performed in triplicates and measured twice. **B:** Inhibition of proliferation by LY. Subconfluent HDF were treated with the indicated concentrations of LY for 3 days. Cell numbers were determined in triplicates. **C:** Comparison of HDF after LY treatment and HDF after reaching replicative senescence. Morphology (left panel, 100x magnification) and detection of senescence associated β-galactosidase at pH 6 (right panel, magnification 200x) using phase contrast microscopy. **D:** Irreversible effect of LY on proliferation of HDF. Cells were treated with 1 µM LY for the indicated periods. After trypsinization cell numbers were determined in triplicates. **E:** FACS analysis of HDF treated with LY. HDF were synchronized by confluence and released by trypsinization. After 24 and 36 hours incubation in media containing 1 µM LY or vehicle cells HDF were collected for FACS analysis. **F:** Rate of DNA-synthesis after LY treatment. BrdU-incorporation was determined after incubation of HDF in 10 µM BrdU for 6 hours.

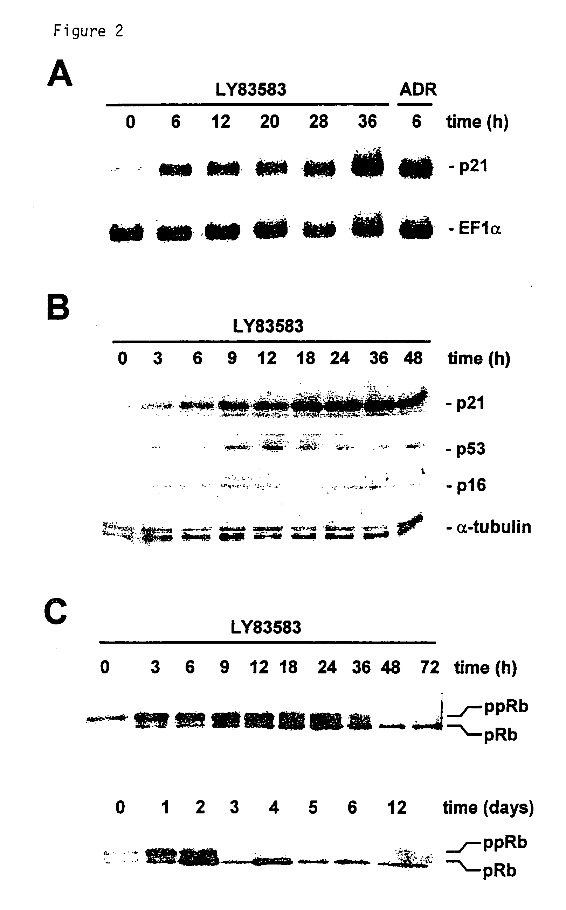

Fig.2 shows the effects of LY on the expression and phosphorylation status of cell cycle regulatory proteins. **A:** Northern blot analysis of LY-treated HDF. Total RNA was isolated from HDF treated with 2 µM LY or 0.2 µg/ml adriamycin for the indicated periods. 7.5 µg of total RNA was loaded per lane. *p21*^{*WAF1*}^{/}^{*CIP1*}^{/}^{*SDI*} and, as a control, *ELF1α* mRNA was detected with ³²P-labeled probes. **B:** Western blot analysis of HDF treated with LY. Lysates were obtained from HDF treated with LY for the indicated periods. 20 µg protein was loaded per lane. After separation on 4-20 % gradient gels and transfer to PVDF membranes, proteins were detected with antibodies against human p21, p53, p16, and as a loading control α-tubulin. **C:** pRb phosphorylation status after treatment with LY. After addition of LY for the indicated periods cell lysates were prepared and proteins were separated on a 7-12 % gradient denaturing PAGE-gel. Abbreviations: ppRb, hyper-phosphorylated pRb; pRb, hypo-phosphorylated pRb.

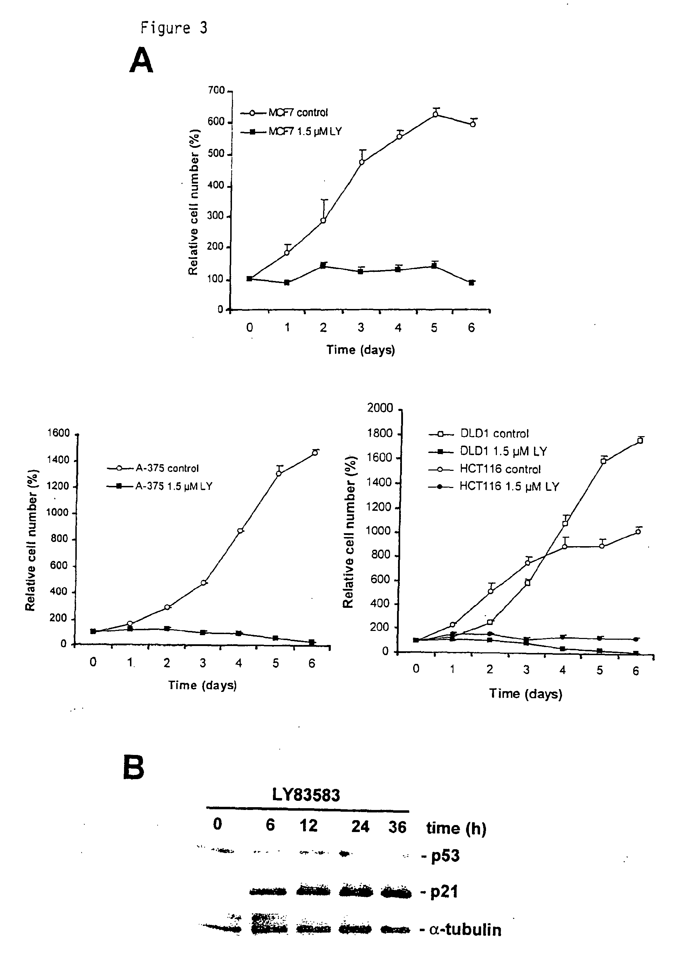

Fig.3 shows the effects of LY on epithelial cancer cells. **A:** Growth-curves of MCF-7, A-375, HCT116, and DLD1 cells. Cells were trypsinized at the indicated time points after addition of LY to the media and cell numbers were determined. **B:** Induction of p21 protein after treatment with LY. HCT116 colorectal cancer cells were treated with 1.5 µM LY for the indicated periods. Cell lysates were prepared and subjected to Western blot analysis with the indicated antibodies.

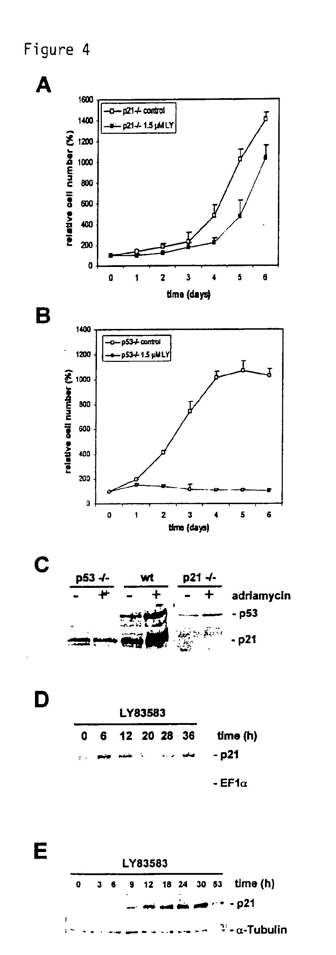

Fig.4 shows the requirement of *p21,* but not p53, for LY-induced cell cycle arrest. **A:** HCT116 colorectal cancer cell line deficient for *p21.* Cells were treated with LY or, as a control, with vehicle (DMSO) and cell numbers were assessed. Measurements were performed in triplicates. **B:** *p53*+/+ and *p53*-/- HCT116. Cells were treated with LY or, as a control, with vehicle (DMSO) and cell numbers were assessed. Measurements were performed in triplicates. **C:** Confirmatory analysis of HCT116-derived knock-out cell lines. Western blot analysis of p53 and p21 protein levels after addition of adriamycin at 0.2 µg/ml for 8 hours. **D:** p53-independent induction of p21 mRNA by LY. Northern blot analysis with RNA isolated at the indicated time-points after addition of 1.5 µM LY to p53-deficient HCT116 cells. **E:** p53-independent induction of p21 protein levels by LY. Western blot analysis with lysates from p53-/- HCT116 cells treated with 1.5 µM LY. Membranes were probed with antibodies specific for p21 and as a loading control α-tubulin.

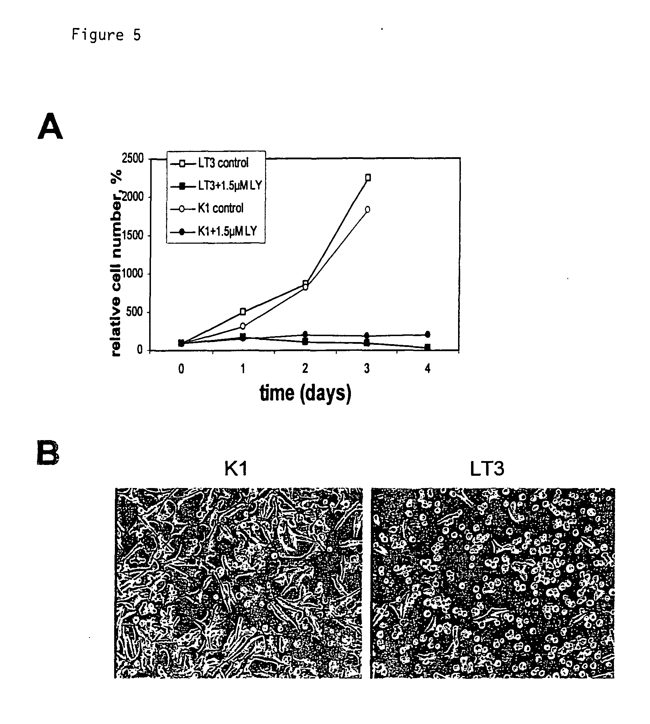

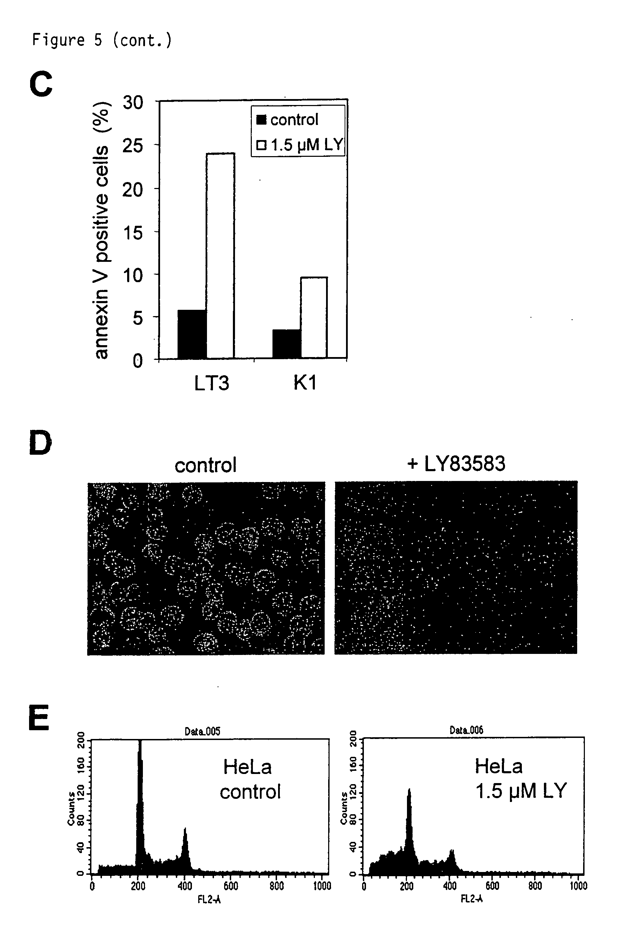

Fig.5 shows the effect of LY on cells with inactivated pRb pathways. **A:** Response of cell lines with lesions in the pRb-pathway to LY. NIH3T3-L1 cells expressing SV40 large T antigen (LT3) and NIH3T3-L1 cells expressing a point mutant of SV40 large T antigen (K1 ) unable to bind to pRb-like proteins were treated with 1.5 µM LY. Cell numbers were determined at the indicated time points in triplicates. **B:** Morphology of NIH3T3-L1 cells after LY treatment. Phase contrast microscopy was performed after incubation of NIH3T3-L1-K1 (K1) and NIH3T3-L1-LT3 (LT3) cells in 1.5 µM LY for 3 days (magnification: 100x). **C:** Quantification of early stages of apoptosis. Control and LY-treated for 48 hours K1 and LT3 cells were subjected to double staining with anti-annexin V and propidium iodide (PI) to discriminate living, early apoptotic, and late apoptotic/necrotic populations and analyzed with flow cytometry. The percentage of PI-negative annexin V-positive cells is shown. **D:** Induction of apoptosis by LY. HeLa cells expressing a histone H2B-eGFP fusion protein (kindly provided by G. Wahl) were treated with 2 µM LY for 48 hours and the chromatin morphology in living cells was analyzed at a wave-length of 495 nm. **E:** Quantification of apoptosis by detection of cells with sub-G1 DNA content. HeLa cells were treated for 60 hours with LY. DNA-content was determined after propidium iodide staining as described in the Examples.

### Examples

This study characterizes the effects of LY on non-transformed primary cells and tumor cells with different lesions in key tumor suppressor pathways. LY represents a new type of non-genotoxic drug which inhibits tumor cell proliferation by induction of p21 and subsequent context dependent activation of cell cycle arrest or apoptosis. The p53 independence of the antiproliferative effect of LY and its ability to induce apoptosis in cells with non-functional RB protein make this substance an interesting prototype for the development of new non-genotoxic cancer therapeutics. Comparative analysis of gene expression profiles of replicative and LY-induced premature senescence suggests the existence of a transcriptional program specific for senescence which is activated irrespective of the inducing signal and presumably represents a safeguard mechanism to prevent proliferation of potentially cancerous cells.

### I. Experimental procedures

### I.1. Cell culture and drug treatments

HDF were obtained from Clonetics and passaged in Dulbecco's modified Eagles medium (DMEM, Gibco) supplemented with 10 % fetal bovine serum (FBS, Sigma). HCT116 were cultured in McCoy's (Gibco) supplemented with 10 % FBS (Sigma). HeLa, HEK293, P16-/-MEFs and NIH3T3-L1 derivatives were kept in DMEM containing 10 % FBS. LY (Calbiochem) was dissolved in DMSO (Sigma) at a concentration of 300 µM (~ 300x solution). As a control, cells were treated with equal volumes of DMSO (< 1 %). The LY concentration was restored in intervals of 24 hours by media exchange.

### I.2. Microarray analysis of gene expression

RNA was isolated using RNAgents reagents (Promega). After mRNA isolation, integrity and enrichment was ensured using Northern blot analysis. 600 ng poly-A mRNA was converted to cDNA with incorporation of Cy3- or Cy5-labeled dNTPs. Hybridization to arrays coated on glass, quality control and normalization was performed by IncyteGenomics (Palo Alto, CA). The 'Human Unigene 1 '-array, used for all microarray analyses of HDF, contained probes for 8,392 annotated genes/EST clusters and 74 non-annotated genes/ESTs (probes are derived from the 5'-cDNA region), whereas the 'Human Drug Target'-array, used for analysis of MCF7 treated with LY, represented 8,031 unique annotated genes/EST clusters and 207 non-annotated genes/EST clusters (probes are derived from the 3'-cDNA region).

### I.3. Northern blot analysis

RNA was isolated using the RNAgents kit (Promega). A Northern probe directed against the 3'-untranslated region of ELF1 α mRNA was generated by PCR using an EST as template and subsequent gel purification. The p21 probe corresponds to the open reading frame of *p21.* A probe corresponding to the 5'-region of sGC-β3 was amplified from HDF-cDNA and subcloned. Hybridizations were performed in QuickHyb, following the manufacturer's instructions (Stratagene).

### I.4. Quantitative real-time PCR

qPCR was performed using the LightCycler and the FastStart DNA Master SYBR Green 1 kit (Roche Applied Science) as previously described (Menssen and Hermeking, 2002). For qPCR of cDNA, primer pairs were designed to generate intron-spanning products of 100-200 bp. Primer sequences were: GUCY1A3-sense 5'-TTCAGAGGAGGCAGCAGG-3' (SEQ ID NO. 1), GUCY1 A3-antisense 5'-GCAACATTCAGCCGTTCAA-3' (SEQ ID NO.2; annealing temperature 62°C, efficiency 1.97); GUCY1 B3-sense 5'-AGGAATCACGCATCAGCC-3' (SEQ ID NO.3), GUCY1 B3-antisense 5'-TATGAGGACGAACCAGCGA-3' (SEQ ID NO.4; annealing temperature 62°C, efficiency 1.94 ).

cDNA was generated using the RevertAid First Strand cDNA synthesis kit (MBI Fermentas). The generation of specific PCR products was confirmed by melting curve analysis and gel-electrophoresis. Each primer pair was tested with a logarithmic dilution of a cDNA mix to generate a linear standard curve (crossing point (CP) plotted vs. log of template concentration), which was used to calculate the primer pair efficiency (E = 10^{(-1/slope)}). Elongation factor 1*α* (*ELF1α*) mRNA was used as an external standard since its expression was not altered significantly in senescent HDF (data not shown). For data analysis, the second derivative maximum method was applied and induction of a cDNA species (geneX) was calculated according to Pfaffl (2001): (E_{geneX} ^{^ΔCP}(confl. _{cDNA - sen.cDNA)} ^{geneX}) / (E_{ELF1α} ^ ^{ΔCP}_{(confl.cDNA - sen.cDNA)} ^{ELF1α}) = fold induction.

### I.5. Measurement of DNA content and apoptosis by flow cytometry

Cell samples (both adherent and floating cells) were harvested by trypsinization, washed once with PBS and fixed in 70 % ethanol for > 2 hours on ice, washed once with PBS and incubated for 30 minutes at room temperature in staining solution containing 50 µg/ml of propidium iodide, 0.2 mg/ml of RNase A and 0.1 % (v/v) Triton X-100 in PBS. Quantification of apoptotic cells was performed using the Annexin V-FITC apoptosis detection kit (Becton Dickinson). Samples were analyzed with a FACScan unit (Becton Dickinson). 1×10⁴ cells were analyzed for each assay. Quantitation was performed using single histogram statistics.

### I.6. Proliferation assay

Cells were seeded in equal numbers in 6-well plates the day before addition of LY in order to ensure uniform cell layers at the start of the experiments. Cells were treated in triplicates with daily exchange of medium containing drug or drug-free vehicle. For each time point cells were trypsinized and cell proliferation was assessed using a Z1 Counter (Coulter).

### I. 7. cGMP assay

Cells of early and late passages were seeded in equal numbers in 6-well plates. 24 hours later SNP (Sigma) was dissolved in complete medium and added to cells to a final concentration of 100 µM. Control cells received the same volume of drug-free medium. After 2 hours of incubation, medium was removed and cells were lysed by addition of 400 µl 0.1 M HCl per well for 10 minutes. cGMP concentration in lysates was determined using a specific competitive immunoassay according to the manufacturer's instructions (Sigma). A plate reader (LAMBDA E, MWG-Biotech) was used to detect the signal at 405 nm. Experiments were performed in triplicates with all samples measured twice.

### I.8. SA-β-gal staining

Cells were fixed by incubation in 0.5 % glutaraldehyde in PBS for 5 minutes at room temperature and stained for SA-ß-galactosidase at pH 6.0 as described by Dimri *et al.,* 1995.

### I.9. Detection of DNA-synthesis

Cells were plated on cell locate glass grids (Eppendorf) and labeled for 6 hours using the 5'-bromo-2'-deoxy-uridine labelling and detection kit I (Roche Applied Sciences). After staining with a FITC-labeled anti-BrdU-antibody, positive cells were detected by fluorescence microscopy.

### I.10. Western blot analysis

Cells were lysed in a lysis buffer composed of 50 mM HEPES pH 7.5, 150 mM NaCl, 1 mM EGTA, 10 % glycerol, 1 % Triton X-100, 100 mM NaF, 10 mM Na₄P₂O₇, 1 µM PMSF, and 1 µM Na₃VO₄. Protein concentration in whole lysates was determined using a Bradford assay (BioRad). 20 µg of lysates diluted with Laemmli-buffer were separated on 12 % SDS-polyacrylamide gels and transferred to Immobilon-P membranes (Millipore). For detection of pRb, separation was performed on a 7-12 % gradient gel. Antibodies against p53 (Pab 1801), p21 (c-19), p16 (c-20) and α-Tubulin (TU-02) were purchased from Santa Cruz Biotechnology. Anti-pRB antibodies (G3-245) were from BD PharMingen. Enhanced chemiluminescence generated by horse radish peroxidase-coupled secondary antibodies was detected with a CF440 imager (Kodak/Perkin Elmer).

### I.11.Microscopy

For phase contrast and β-gal staining an Axiovert 25 microscope (Zeiss) equipped with a HyperHAD CCD camera (Sony) and ImageBase software (Kappa) was used. *In vivo* expression of histone-H2B-eGFP was documented with a Photometrics CooISNAP-HQ CCD camera (Roper Scientific) on an inverted Axiovert 200M microscope (Zeiss) using the Metamorph software (Universal Imaging).

### II. Results

### II. 1. Microarray analysis of senescence-specific gene expression

We employed microarray analysis in order to identify genes and pathways involved in the induction and maintenance of replicative senescence. Primary human diploid fibroblasts (HDF) were cultivated for ~78 population doublings until proliferation of all cells ceased due to replicative senescence. At this point, HDF showed characteristic signs of senescence: cellular enlargement, SA-β-galactosidase staining at pH 6 (Figure 1 C) and terminal arrest in the presence of high serum concentration (mainly in the G₁-phase as determined by FACS analysis, data not shown). mRNA was isolated from senescent HDF and subjected to analysis with a microarray, which allows detection of 8,392 annotated cDNAs. As a reference, a microarray analysis was performed with RNA derived from presenescent HDF, which had been arrested in the G₁-phase by confluence at population doubling 12. This state was selected to avoid a comparison of non-proliferating senescent HDF with proliferating early passage HDF, which would presumably result in the detection of a large number of differences in gene expression secondary to growth-arrest. Several genes previously identified as consistently up- or down-regulated during senescence of HDF were detected as regulated in a similar fashion in the system employed here (Table 1 A): e.g. *PAI-1* was previously detected as up-regulated in senescent HDF (West *et al.,* 1996; Ly *et al.,* 2000; Shelton *et al.,* 1999) and was induced 5.9 fold in senescent HDF in this study (Table 1 A). Among the genes repressed during senescence were four genes encoding enzymatic components of the cGMP-pathway (Table 1 B): soluble guanylate cyclase *β*3 (sGC-β3), sGC-α3 and cGMP-dependent protein kinase I and II. Differential regulation of sGC-α3 and sGC-β3 during senescence was confirmed by quantitative real-time PCR (Table 1). Down-regulation of sGC-α3 during senescence was also detected using Northern blot analysis (data not shown). sGC-α3 and sGC-β3 represent the large and small subunit of soluble guanylate cyclase, which converts GTP to 3 ',5 '-cyclic GMP and pyrophosphate (Giuili *et al.,* 1992). Interestingly, both genes are localized in near proximity on chromosome 4q31.3-q33 (Giuili *et al.,* 1993), which could provide the basis for the co-regulation observed here.

### II.2. Induction of senescence by 6-anilino-5,8-quinolinequinone

The co-ordinated repression of two genes encoding the subunits of soluble guanylate cyclase, which generates cGMP in response to signals as e.g. extra-cellular NO (reviewed in Hofmann *et al.,* 2000), suggested, that senescent cells may have an attenuated response to NO-donors as sodium nitroprusside (SNP). Indeed, exposure to SNP did not result in elevated cGMP levels in senescent HDF, whereas early passage HDF showed a three-fold increase in cGMP levels after addition of SNP (Fig. 1 A). In order to specifically inhibit the cGMP-signalling-pathway, we employed a competitive inhibitor of soluble guanylate cyclase: 6-anilino-quinoline-5,8-quinone (LY 83583, referred to as LY hereafter), with an IC₅₀ of 2 µM (Fleisch *et al.*, 1984). Addition of 1 µM LY HDF significantly inhibited the SNP-induced synthesis of cGMP by 60 % (Fig.1A), showing that LY had the expected inhibitory effect on guanylate cyclase.

We next asked, whether addition of LY would affect the proliferation of early passage HDF, as we would expect according to our working hypothesis described above. The effect of LY was tested at a concentration ranging from 0.25 to 1.5 µM (Figure 1B). Indeed, treatment of early passage fibroblasts with a concentration of 1 µM of LY was sufficient to completely inhibit proliferation, and the presence of 250 nM LY led to a 50 % reduction in proliferation of HDF after 3 days (Figure 1 B). Strikingly, the inhibition of proliferation of early passage HDF by LY was accompanied by morphological changes characteristic of senescence and by SA-β-galactosidase staining at pH 6 (Figure 1 C). Consistent with the induction of cellular senescence, inhibition of proliferation by LY was irreversible after a treatment period of more than 2 days (Figure 1 D). Extended treatment of HDF with LY for 8 days did not cause any obvious toxicity but resulted in stable inhibition of proliferation (Figure 1 D). In order to characterize the cell cycle arrest induced by LY, HDF were synchronized in the G1-phase by confluence (91.2 % G₁- and 2 % S-phase) and then released by trypsinization (Figure 1 E): untreated cells entered S-phase at a high percentage. Addition of LY almost completely blocked the entry into S-phase of HDF, suggesting that LY acts during the G1-phase of the cell cycle. Furthermore, addition of 1 µM LY to synchronized HDF caused complete inhibition of DNA synthesis as determined by BrdU incorporation (Figure 1 F).

### II.3. Microarray analysis of LY-regulated genes

In order to identify the down-stream mediators of the LY-induced cell cycle arrest and senescence, a microarray analysis of LY-treated HDF was performed with the same arrays used for the analysis of replicative senescence in HDF described above. RNA was isolated 36 hours after exposure of early passage HDF to LY. The control RNA was isolated from confluent early passage HDF. In HDF, which had reached replicative senescence, 216 transcripts were induced significantly, whereas 272 mRNAs were induced after LY treatment. Repression was observed for 266 mRNAs in HDF undergoing replicative senescence, whereas 294 mRNAs were repressed after LY treatment. There was a substantial overlap in the differentially expressed genes observed during replicative senescence and during LY-induced senescence, with 114 transcripts differentially regulated in a similar manner (Table 2). Among these were genes, which had been previously identified as differentially regulated during replicative senescence: *PAI-1,* matrix *metalloproteinase 10*, *fibrillin, cdc25b*, *cyclin D1, fibromodulin* and *osf-2* (Shelton *et al.,* 1999; Ly *et al.,* 2000). However, most of the genes identified here represent new additions to the growing number of genes identified as components of the senescence program. Supporting this notion, many of the co-regulated genes have functions, which may contribute to the phenotype of terminally arrested cells. E.g. down-regulation of the PDGF-receptor α and β chains may contribute to the refractory state of senescent cells, which do not respond to mitogenic stimulation with growth factors (Table 2). The changes in expression observed in genes encoding components of the cytoskeleton (e.g. α1-tubulin, β2-tropomyosin, y-filamin C, α-actinin, α-tubulin) may contribute to the flattened and enlarged shape characteristic of senescent HDF (Figure 1C).

### II.4. Rapid induction of p21^{WAF1}^{/}^{CIP1}^{/}^{SDI} by _{LY}

Among the genes induced by both, replicative senescence as well as LY, was *p21*^{*WAF1*}^{/}^{*CIP1*}^{/}^{*SDI*} (Table 2), which encodes an inhibitor of cyclin dependent kinases (cdk). Cdk/cyclin complexes drive cell cycle progression and proliferation by phosphorylation of key substrates (reviewed in Nurse, 2000). Since induction of *p21* could potentially explain the anti-proliferative effect of LY, we analyzed the connection between LY and *p21* in more detail. 6 hours after treatment with LY, the levels of *p21* mRNA in HDF were similar to the levels observed 6 hours after addition of adriamycin as shown by Northern blot analysis (Figure 2A). However, after treatment with LY, the level of p53 protein, which consistently increases after generation of DNA damage and mediates transactivation of p21, did not increase (Figure 2B), indicating that LY does not induce DNA-damage. The induction of *p21* mRNA by adriamycin demonstrates, that the signalling pathways necessary for p53 activation were still intact in the HDF employed (Figure 2A). Induction of p21 protein was detectable between 3 and 6 hours after addition of LY, whereas the cdk-inhibitor p16^{INK4A} was not induced even after 48 hours of treatment with LY (Figure 2B). This result was unexpected, since p *16*^{*INK4A*} is commonly induced in scenarios, which lead to premature senescence, as e.g. ectopic expression of an activated *ras* gene (Serrano *et al.,* 1997) or sub-optimal cell culture conditions (Ramirez *et al.,* 2001).

The major down-stream targets of p21 are the pocket proteins: the retinoblastoma tumor suppressor protein pRb and its homologs p 107 and p 130. p21-mediated inhibition of G1/S-phase associated cdk/cyclin-complexes, as cdk2/cyclin A, leads to hypo-phosphorylation of pocket proteins, which allows their subsequent binding and inhibition of E2F transcription factors. The association between pRb and E2Fs results in inhibition of G₁/S progression and proliferation (reviewed in Weinberg, 1995). Consistent with the induction of *p21* by LY, the phosphorylation status of pRb changed rapidly after addition of LY: after only 6 hours the ratio of phosphorylated to hypo-phosphorylated, active pRb was clearly shifted towards the hypo-phosphorylated form and by 19 hours pRb was almost completely hypo-phosphorylated (Figure 2C). Even after extension of the LY treatment to 12 days, the pRb hypo-phosphorylation was not reversed (Figure 2C). These results show, that LY inhibits proliferation by inducing p21 to levels, which are sufficient to inhibit cdk activity and activate the cell cycle inhibitory function of pRb.

### II.5. Effect of LY on cancer cell proliferation

In order to determine, whether LY may be useful to terminate cancer cell proliferation, the proliferation of colorectal cancer cell lines (HCT116, DLD1) and a breast cancer derived cell line (MCF7) were analyzed after addition of LY: all cell lines showed complete cessation of cell proliferation (Figure 3A). Using microarray analysis 360 genes were identified as differentially regulated by LY in MCF7 cells (Table 3). However, the amount of overlap with genes differentially expressed in LY-treated HDF could not be determined, since the array employed for the analysis of gene expression in MCF7 differed substantially from the arrays used for HDF before. However, *p21* was among the genes induced by LY in MCF7 cells, suggesting that *p21* may be involved in the LY-induced arrest in MCF7 and other epithelial cancer cells. Consistent with this assumption, western blot analysis of HCT116 cells showed induction of p21 after addition of LY (Figure 3B).

### II.6. Requirement of p21, but not p53, for LY-induced cell cycle arrest

In order to determine, whether induction of *p21* is essential for LY-induced cell-cycle arrest, *p21*-deficient HCT116 colorectal cancer cells, generated via homologous recombination by Waldman *et al.* (1996), were analyzed (Figure 4A): *p21*-deficient HCT1 16 were resistant to cell-cycle inhibition by LY. Compared to cells treated with vehicle alone, LY-treatment resulted in an initial delay of proliferation in *p21*-/- HCT116, suggesting other immediate, *p21*-independent effects of LY on HCT116 cells. However, after 5 days of LY treatment, the *p21*-deficient HCT116 cells had regained exponential proliferation, whereas wild-type HCT116 cells showed complete cessation of proliferation. These results show that *p21* is required for the effects of LY on the cell cycle.

Consistent with the absence of p53 accumulation in LY-treated HDF, p53-deficient HCT116 cells engineered by Bunz *et al.* (1999) showed a complete block of proliferation after LY addition (Figure 4B). The identity of the 3 different HCT116 cell lines employed in this study was confirmed by treatment with adriamycin, which induces DNA double-strand breaks (Figure 4C). As expected, only wildtype HCT116 cells showed induction of p21 protein after exposure to adriamycin (Figure 4C). The levels of *p21* mRNA and protein of p53-/- HCT116 after treatment cells with LY were induced with kinetics similar to those observed in HDF (Figure 4D and E). These results exclude p53 and thereby induction of DNA damage as mediators of cell cycle arrest by LY and induction of *p21* expression. Furthermore, LY-treatment did not lead to induction of the cdk-inhibitors p27 and p15 (data not shown). In addition, proliferation of *p16*^{*INK4A*}-deficient mouse embryo fibroblasts (MEF) was inhibited by LY, showing that p *16*^{*INK4A*} is not required for the effects of LY on the cell cycle (data not shown). Taken together, these results show, that the inhibitory effects of LY on proliferation are mediated through induction of *p21.*

### II.7. Conversion of LY-induced arrest to apoptosis in pRb-negative cells

Since the anti-proliferative effect of p21 is mediated through the pRb-pathway (reviewed in Weinberg, 1995), we analyzed the response of cells with non-functional pRb to treatment with LY. Isogenic NIH3T3-L1 cell lines stably expressing either SV40 large T antigen or a SV40-large T antigen point mutant (K1) deficient in binding and inactivating pRb-like pocket-proteins (described in Hermeking *et al.,* 1994) were treated with LY: most NIH3T3-L1 cells expressing the K1-mutant showed inhibition of proliferation, whereas NIH3T3-L1 cells expressing wildtype SV40 large T antigen underwent apoptosis (Figure 5A and B). Induction of apoptosis by LY was further quantified using annexin V staining (Figure 5C).

In order to extend these observations to human cancer cells, the cell lines HeLa and HEK293 were analyzed. The cervical cancer cell line HeLa expresses the human papilloma virus (HPV) 18 encoded E7 protein, which binds to and inactivates pocket proteins (pRb, p107, p130). In HEK293 cells, the adenoviral E1A protein performs analogous functions. HeLa and HEK293 cells did not arrest after treatment with LY, but instead, underwent cell death, which was accompanied by cell shrinkage suggesting apoptosis (data not shown). HeLa cells expressing a histone H2B-eGFP-fusion protein allowed to confirm the induction of apoptosis by LY (Figure 5D): treatment with LY led to chromatin condensation, which is characteristic of apoptosis. Furthermore, analysis of a larger cell population by flow-cytometry revealed a substantial increase of cells with a sub-G₁ DNA content after addition of LY (Figure 5E).

### III. Discussion

The results described here demonstrate, that LY induces cellular senescence in HDF and cell cycle arrest in several human and murine cell types. One requirement for these effects of LY is the transcriptional induction of the cdk-inhibitor p21, since *p21*-deficient cells are resistant to LY-induced arrest. Furthermore, in the absence of functional pocket proteins (pRb, p107, p130), which are downstream effectors of p21 function, cell cycle arrest is converted to induction of apoptosis in a p53-independent manner. We assume, that in this case apoptosis is a cellular response to conflicting signals: negative signals due to LY-induced p21 and resulting cdk-inhibition versus cell cycle driving signals due to the lack of active pocket proteins, which allow high levels of active E2F transcription factors.

The induction of *p21* is not mediated through the DNA-damage/p53-pathway, since p53-deficient cells also show up-regulation of *p21* by LY and subsequent cell cycle arrest. These features of LY indicate that it may be a useful agent for treatment of cancer. Many tumor cells have lost pRb function, either through mutation in pRb (Horowitz *et al.,* 1990) or through expression of viral proteins. E.g. in cervical cancer expression of E7 by HPV16/18 leads to inactivation of pRb (reviewed in zur Hausen, 2001). In HPV16/18-infected cells treatment with LY would presumably induce apoptosis. Since LY induces *p21*, cell cycle arrest, and apoptosis in a p53-independent manner, it may proof useful for the treatment of cancers harboring *p53* mutations, which represent more than half of all human cancers (reviewed in Hollstein *et al.,* 1999).

In order to induce senescence in early passage cells, we decided to inhibit the cGMP-pathway, since components of this pathway were transcriptionally suppressed during senescence. For this purpose LY was selected, since it specifically inhibits soluble guanylate cyclase (Mulsch *et al.*, 1988). LY was first described as an inhibitor of leukotriene release (Fleisch *et al.,* 1984). Later it was found to lower cGMP levels (Schmidt et *al*., 1985) by inhibiting soluble guanylate cyclase (Mulsch *et al.,* 1988). In the experiments presented here LY was able to mimic cellular senescence in primary human fibroblasts and induced a cell cycle arrest in several cancer cell lines. However, it is unclear, whether inhibition of cGMP generation is the only pathway by which LY achieves its effects. It has been proposed that the quinone structure of LY may lead to the generation of superoxide anions (Cherry *et al.,* 1990). However, a recent report, employing the colorectal cancer cell line HCT116 and isogenic *p21-* or p53-deficient cells also analyzed in this study, showed, that reactive oxygen species (ROS) generated by hyperoxic conditions induce expression of *p21* in a p53-dependent manner (Helt *et al.,* 2001). Furthermore, proliferation of HCT116 cells is inhibited by hyperoxic conditions in a p21- and p53-independent manner (Helt *et al.,* 2001). In contrast to induction of *p21* by ROS, induction of *p21* by LY was not mediated by p53. Furthermore, inhibition of proliferation by LY was dependent on *p21.* These results suggest, that LY does not act by generating ROS, which in turn mediate cell cycle arrest or senescence.

Furthermore, it is important to analyze whether LY or derived substances can be used to prevent tumor growth *in vivo* using animal models. The results provided thereby may serve as a basis for further characterization and optimization of LY-derived drugs as tumor therapeutics. An obvious advantage of LY is its ability to interfere with cellular proliferation without inducing DNA-damage. Commonly used chemotherapeutics, like adriamycin, generate DNA damage and thereby selectively induce apoptosis in cancer cells, which lack cell cycle check points (reviewed in Hartwell *et al.,* 1997). However, DNA damaging substances cause major side effects on proliferating tissues (Batchelor, 2001) and may induce further mutations in pre-cancerous and healthy cells, which lead to secondary cancer (Felix *et al.,* 1998).

The regulation of more than one hundred senescence-specific genes by LY shows, that treatment with LY activates a transcriptional program, which is analogous to the program induced after HDF have reached replicative senescence. Therefore, the genes shown in Table 2 represent a transcriptional program characteristic for senescence of HDF.

The strategy underlying the present invention, i.e. identification of pathways, which are repressed during senescence and their inhibition in tumor cells by synthetic drugs may also proof useful for the identification of other potential targets for effective inhibitors of tumor cell proliferation.

### Literature

Bast, R.C., Kufe, D.W., Pollock, R.E., Weichselbaum, R.R.; Holland, J.F. and Frei, E., eds.(2000). Cancer Medicine (BC Decker Inc.).
Batchelor, D. (2001). Hair and cancer chemotherapy: consequences and nursing care-a literature study. Eur. J. Cancer Care *10*,147-163.
Brown, J.P., Wei, W. and Sedivy, J.M. (1997). Bypass of senescence after disruption of p21CIP1/WAF1 gene in normal diploid human fibroblasts. Science *277*, 831-834.
Bunz, F., Dutriaux, A., Lengauer, C., Waldman, T., Zhou, S., Brown, J.P., Sedivy, J.M., Kinzler, K.W. and Vogelstein, B. (1998). Requirement for p53 and p21 to sustain G2 arrest after DNA damage. Science *282*, 1497-1501.
Campisi, J. (2001). Cellular senescence as a tumor-suppressor mechanism. Trends Cell Biol. *11*, 27-31.
Chang, B.D., Xuan, Y., Broude, E.V., Zhu, H., Schott, B., Fang, J. and Roninson, I.B. (1999). Role of p53 and p21 waf1/cip1 in senescence-like terminal proliferation arrest induced in human tumor cells by chemotherapeutic drugs. Oncogene *26*, 4808-4818.
Cherry, P.D., Omar, H.A., Farrel, K.A., Stuart, J.S. and Wolin, M.S. (1990). Superoxide anion inhibits cGMP-associated bovine pulmonary arterial relaxation. Am. J. Physiology *259*, 1056-1062
Cristofalo, V.J., Volker, C., Francis, M.K. and Tresini, M. (1998). Age-dependent modifications of gene expression in human fibroblasts. Crit. Rev. Eukaryot. Gene Expr. *8*, 43-80.
Dellambra, E., Golisano, O., Bondanza, S., Siviero, E., Lacal, P., Molinari, M., D'Atri, S. and De Luca, M. (2000). Downregulation of 14-3-3sigma prevents clonal evolution and leads to immortalization of primary human keratinocytes. J Cell Biol. *149*, 1117-1130.
Di Leonardo, A., Linke, S.P., Clarkin, K. and Wahl, G.M. (1994). DNA damage triggers a prolonged p53-dependent G1 arrest and long-term induction of Cip1 in normal human fibroblasts. Genes Dev. *8*, 2540-2551.
Dimri, G.P., Lee, X., Basile, G., Acosta, M., Scott, G., Roskelley, C., Medrano, E.E., Linskens, M., Rubelj, I., Pereira-Smith, O., *et al.* (1995). A biomarker that identifies senescent human cells in culture and in aging skin in vivo. Proc. Natl. Acad. Sci. USA. *92*, 9363-9637.
Green, D.R. and Evan, G.I. (2002). A matter of life and death. Cancer Cell *1*, 19-30.
Giuili, G., Roechel, N., Scholl, U., Mattei, M.-G. and Guellaen, G. (1993). Colocalization of the genes coding for the alpha-3 and beta-3 subunits of soluble guanylyl cyclase to human chromosome 4 at q31.3-q33. Hum. Genet. *91*, 257-260.
Giuili, G., Scholl, U., Bulle, F. and Guellaen, G. (1992). Molecular cloning of the cDNAs coding for the two subunits of soluble guanylyl cyclase from human brain. FEBS Lett. *304*, 83-88.
Felix, C.A. (1998). Secondary leukemias induced by topoisomerase-targeted drugs. Biochim. Biophys. Acta. *1400*, 233-255.
Fleisch, J.H., Haisch, K.D., Spaethe, S.M., Rinkema, L.E., Cullinan, G.J., Schmidt, M.J. and Marshall, W.S. (1984). Pharmacologic analysis of two novel inhibitors of leukotriene (slow reacting substance) release. J Pharmacol Exp Ther. *229*, 681-689.
Hartwell, L.H., Szankasi, P., Roberts, C.J., Murray, A.W. and Friend, S.H. (1997). Integrating genetic approaches into the discovery of anticancer drugs. Science *278*, 1064-1068.
Hayflick, L. and Moorehead, P.S. (1961). Exp. Cell Res. *25*, 585-621.
Helt, C.E., Rancourt, R.C., Staversky, R.J. and O'Reilly, M.A. (2001). p53-dependent induction of p21(Cip1/WAF1/Sdi1) protects against oxygen-induced toxicity. Toxicol Sci. *63*, 214-222.
Hermeking, H., Wolf, D.A., Kohlhuber, F., Dickmanns, A., Billaud, M., Fanning, E. and Eick, D. (1994). Role of c-myc in simian virus 40 large tumor antigen-induced DNA synthesis in quiescent 3T3-L1 mouse fibroblasts. Proc. Natl. Acad. Sci. USA. *91*, 10412-10416.
Hofmann, F., Ammendola, A. and Schlossmann, J. (2000). Rising behind NO: cGMP-dependent protein kinases. J. Cell Sci. *113*, 1671-1676.
Hollstein, M., Hergenhahn, M., Yang, Q., Bartsch, H., Wang, Z.Q. and Hainaut, P. (1999). New approaches to understanding p53 gene tumor mutation spectra. *Mutat.* Res. *431*, 7-12
Horowitz, J.M., Park, S.H., Bogenmann, E., Cheng, J.C., Yandell, D.W., Kaye, F.J., Minna, J.D., Dryja, T.P. and Weinberg, R.A. (1990). Frequent inactivation of the retinoblastoma anti-oncogene is restricted to a subset of human tumor cells. Proc. Natl. Acad. Sci. USA. *87*, 2775-2779.
Ly, D.H., Lockhart, D.J., Lerner, R.A. and Schultz, P.G. (2000). Mitotic misregulation and human aging. Science *287*, 2486-2492.
Menssen, A. and Hermeking, H. (2002). Characterization of the c-MYC-regulated transcriptome using SAGE: Identification and characterization of c-MYC-target genes. Proc. Natl. Acad. Sci. USA., *in press*
Mulsch, A., Busse, R., Liebau, S. and Forstermann, U. (1988). LY 83583 interferes with the release of endothelium-derived relaxing factor and inhibits soluble guanylate cyclase. J. Pharmacol. Exp. Ther. *247*, 283-288.
Noda, A., Ning, Y., Venable, S.F., Pereira-Smith, O.M. and Smith, J.R. (1994). Cloning of senescent cell-derived inhibitors of DNA synthesis using an expression screen. Exp Cell Res. *211*, 90-98.
Nurse, P. (2000). A long twentieth century of the cell cycle and beyond. Cell *100,* 71-78.
Pfaffl, M.W. (2001). A new mathematical model for relative quantification in real-time RT-PCR. Nucleic Acids Res. *29*, 2003-2007.
Ramirez, R.D., Morales, C.P., Herbert, B.S., Rohde, J.M., Passons, C., Shay, J.W. and Wright, W.E. (2001). Putative telomere-independent mechanisms of replicative aging reflect inadequate growth conditions. Genes Dev. *15*, 398-403.
Shay, J.W. and Wright, W.E. (2000). Hayflick, his limit, and cellular ageing. Nat. Rev. Mol. Cell Biol. *1*, 72-76.
Serrano, M. and Blasco, M.A. (2001). Putting the stress on senescence. Curr. Opin. Cell. Biol. *13*, 748-753.
Serrano M., Lin A.W., McCurrach M.E., Beach D. and Lowe S.W. (1997). Oncogenic ras provokes premature cell senescence associated with accumulation of p53 and p16INK4a. Cell *88*, 593-602.
Schmidt, M.J., Sawyer, B.D., Truex, L.L., Marshall, W.S. and Fleisch, J.H. (1985). LY: an agent that lowers intracellular levels of cyclic guanosine 3',5'-monophosphate. J Pharmacol Exp Ther. *232*, 764-769.
Shelton, D.N., Chang, E., Whittier, P.S., Choi, D. and Funk, W.D. (1999). Microarray analysis of replicative senescence. Curr. Biol., *9*, 939-945.
Tyner, S.D., Venkatachalam, S., Choi, J., Jones, S., Ghebranious, N., Igelmann, H., Lu, X., Soron, G., Cooper, B., Brayton, C., Hee Park, S., Thompson, T., Karsenty, G., Bradley, A. and Donehower, L.A. (2002). p53 mutant mice that display early ageing-associated phenotypes. Nature *415*, 45-53.
Waldman, T., Lengauer, C., Kinzler, K.W. and Vogelstein, B. (1996). Uncoupling of S phase and mitosis induced by anticancer agents in cells lacking p21. Nature *381*, 713-716.
Weinberg, R.A. (1995). The retinoblastoma protein and cell cycle control. Cell *81*, 323-830.
West, M.D., Shay, J.W., Wright, W.E. and Linskens, M.H. (1996). Altered expression of plasminogen activator and plasminogen activator inhibitor during cellular senescence. Exp. Gerontology, *31*, 175-193.
zur Hausen H. (2001). Oncogenic DNA viruses. Oncogene *20*, 7820-7830.

**Table 1**

| **A** | | | |
|---|---|---|---|
| **symbol** | **description** | **differential expression** | |
| **PAI1** | plasminogen activator inhibitor I | +5.9 | |
| **PAI2** | plasminogen activator inhibitor II | +4.7 | |
| **MMP10** | matrix metalloproteinase 10 | +3.1 | |
| **p21** | cyclin-dependent kinase inhibitor 1A | +2.0 | |
| **CTGF** | connective tissue growth factor | +3.7 | |
| **CCND1** | cyclin D1 | +5.0 | |
| **PK3** | pyruvate kinase | +5.0 | |
| **ID2** | inhibitor of DNA binding 2, ID2 | -2.0 | |

| **B** | | | |
|---|---|---|---|
| **symbol** | **description** | **differential expression** | **qPCR (st. dev.)** |
| **GUCY1B3** | soluble guanylate cyclase 1, beta 3 | -7.8 | -9.4(0.7) |
| **GUCY1A3** | soluble guanylate cyclase 1, alpha 3 | -2.5 | -2.2 (0.4) |
| **PRKG2** | cGMP-dependent protein kinase II | -9.7 | |
| **PRKG1** | cGMP-dependent protein kinase I | -2.5 | |

**Table 2**

| **Gene name (functional class)** | **rapl.sen. fold** | **LY fold** |
|---|---|---|
| Cell cycle | | |
| wee1+ homolog | -4.2 | -3.2 |
| ® cell division cycle 25B *⃝ | -2.3 | -3.8 |
| cyclin G2 | -1.7 | -1.9 |
| checkpoint suppressor 1 | -1.8 | -2.2 |
| cdc-like kinase 1 | -2.2 | -2.8 |
| cydinD1 *⃝ | +5.0 | +2.0 |
| cyclin-dependent kinase inhibitor 1A (p21, Cip1) | +2.0 | +3.0 |
| cyclin-dependent kinase 7 | +1.7 | +1.6 |
| | | |

| **Transcription** | | |
|---|---|---|
| transcription factor 8 | -1.8 | -2.2 |
| Cbp/p300-interacting transactivator (CITED2) | +3.0 | +2.3 |
| TBP-associated factor, RNA pol II, G, 32kD | +1.4 | +1.8 |
| zinc finger protein 238 | -5.5 | -5.6 |
| SWI/SNF rel., matrix assoc., reg.of chromatin 2 | -3.5 | -2.8 |
| ribonuclease, RNase A family, 4 | -4.8 | -7.8 |
| RNA hellcase-related protein | +3.2 | +5.5 |
| c-maf | -4.8 | -2.8 |
| microphthalmia-associated transcription factor | -2.1 | -2.5 |
| | | |

| **Signalling** | | |
|---|---|---|
| PDGF receptor-like (PDGFRL) | -2.9 | -3.0 |
| PDGF receptor, alpha | -2.0 | -2.5 |
| PDGF receptor, beta | -2.4 | -2.7 |
| guanylate cyclase 1, soluble, beta 3 | -7.8 | -3.9 |
| mitogen-activated protein kinase 7 | -1.9 | -2.2 |
| B-factor, properdin | -2.5 | -5.0 |
| glutamate receptor, ionotropic, AMPA 1 | -2.8 | -4.3 |
| TGFb receptor III (betaglycan, 300kD) | -3.8 | -5.1 |
| purinergic receptor P2X, lig.-gated ion channel, 4 | -1.3 | -1.8 |
| prostaglandin F receptor (FP) | -2.1 | -2.2 |
| IGFBP 5 | -16.7 | -10.1 |
| interleukin 1 receptor, type I | -4.9 | -2.2 |
| mitogen-activated protein kinase kinase 5 | -1.8 | -2.0 |
| Interleukin 11 receptor, alpha | -2.9 | -2.1 |
| connective tissue growth factor | +3.7 | +5.0 |
| fibroblast growth factor 2 (basic) | +3.0 | +3.0 |
| annexin A2 | +4.1 | +2.3 |
| adenytyl cyclase-associated protein | +2.0 | +2.0 |
| regulator of G-protein signalling 4 | +1.6 | +5.7 |
| neurotrophic tyrosine kinase, receptor, type 1 | +2.5 | +2.8 |
| SHC 1 | +1.9 | +1.8 |
| regulator of G-protein signalling 4 | +1.6 | +5.7 |
| osteoblast specific factor 2 (fasciclin I-like) *⃝ | -59.9 | -3.3 |
| stromal cell-derived factor 1 | -19 | -2.2 |
| | | |

| **Metabolism** | | |
|---|---|---|
| mannosidase, alpha, class 1A, member 1 | -4.2 | -4.0 |
| alcohol dehydrogenase 1A (class I), a | -3.6 | -3.6 |
| glucosidase, alpha; acid | -2.6 | -2.8 |
| superoxide dismutase 2, mitochondrial | -2.0 | -2.8 |
| phosphatidic add phosphatase type 2B | -7.1 | -6.5 |
| short-chain dehydrogenase/reductase 1 | -2.1 | -3.5 |
| prostaglandin 12 (prostacydin) synthase | -2.1 | -2.3 |
| acid ceramidase | -1.9 | -25 |
| selenophosphate synthetase | -2.4 | -2.2 |
| fucosyltransferase 8 | -1.9 | -2.6 |
| phospholipid scramblase 1 | -2.3 | -2.1 |
| pyruvate kinase | +5.0 | +2.1 |
| UDP-N-acteylglucosamine pyrophosphorylase 1 | +2.2 | +4.1 |
| prostaglandin-endoperoxide synthase 2 (COX2) | +2.1 | +1.8 |
| sialic acid synthase | +2.0 | +1.9 |

| **Extracellular Matrix** | | |
|---|---|---|
| fibulin 2 | -3.4 | -3.4 |
| fibrillin 2 *⃝ | -2.2 | -4.8 |
| fibromodulin *⃝ | -2.2 | -3.8 |
| lumican | -2.2 | -4.8 |
| matrix metalloproteinase 1 | -3.3 | -2.6 |
| matrix metalloprotelnase 2 | -3.9 | -2.4 |
| matrix metalloproteinase 10 (stromelysin 2) *⃝ | +2.3 | +2.9 |
| matrix metalloproteinase 15 | -8.9 | -3.6 |
| matrix Gla protein | -4.8 | -3.1 |
| adducin 3, gamma | -4.1 | -2.6 |
| dermatopontin | -2.0 | -2.9 |
| procollagen C-endopeptidase enhancer | -2.5 | -3.3 |
| cathepsin O | -2.0 | -3.2 |
| cathepsin F | -2.0 | -2.7 |
| protein geranylgeranyltransferase type I, b | -2.0 | +3.2 |
| complement component 1,r subcomponent | -15.7 | -5.2 |
| complement component 1, subcomponent | -9.8 | -5.4 |
| limbic system-associated membrane protein | -1.8 | -3.9 |
| protein S, alpha | -5.7 | -2.7 |
| thrombospondin 2 | -2.0 | -2.6 |
| carboxypeptidase Z | -2.4 | -2.7 |
| glypican 3 | -32.1 | -3.1 |
| PAI-1 *⃝ | +5.9 | +12.6 |
| coagulation factor III | +4.3 | +2.3 |
| metallothlonein 1L | +2.9 | +5.0 |
| | | |

| **Cell shape and motillty** | | |
|---|---|---|
| laminin, alpha 2 | -1.9 | -2.2 |
| microfibrillar-associated protein 4 | -4.0 | -5.6 |
| microfibrillar-associated glycoprotein-2 | -3.6 | -2.0 |
| tropomyosin 2, beta | +3.7 | +2.1 |
| filamin C, gamma | +1.9 | +3.3 |
| tubulin, alpha 1 | +1.8 | +4.3 |
| actin, gamma 1 | +2.2 | +3.7 |
| actinin, alpha 1 | +3.8 | +1.9 |
| actin, beta | +2.5 | +2.8 |
| moesin | +2.6 | +2.4 |
| ARP3 (actin-related protein 3, yeast) | +3.1 | +2.5 |
| | | |

| **Other functions** | | |
|---|---|---|
| cysteine-rich, angiogenic inducer, 61 | +4.1 | +5.5 |
| BRCA1 associated protein | +3.8 | +4.7 |
| heat shock 70kD protein 4 | +1.9 | +1.9 |
| heat shock 70kD protein 8 | +1.9 | +3.7 |
| glioma pathogenesis-related protein | +2.7 | +2.3 |
| butyrophilin, subfamily 3, member A2 | -2.2 | -2.8 |
| glycoprotein A repetitions predominant | -2.1 | -3.0 |
| thioredoxin reductase 1 | +2.2 | +7.8 |
| clusterin | -2.3 | -3.1 |
| upregulated by 1,25-dihydroxyvitamin D-3 | -3.6 | -3.3 |
| selenium binding protein 1 | -2.3 | -2.9 |
| arginyl-tRNA synthetase | +2.0 | +1.8 |
| ubiquitin carboxyl-terminal esterase L1 | +2.1 | +2.0 |
| Fc fragment of IgG, receptor transporter, alpha | -2.6 | -2.8 |
| retinoic acid receptor responder 2 | +2.0 | -2.3 |
| PEG1 | -2.1 | -2.5 |
| latent TGFß binding protein 4 | -11.2 | -2.1 |
| matrilin 2 | -3,9 | -1,9 |
| carboxypeptidase E | -2,4 | -2.0 |
| TGFB inducible early growth response 2 | -1,8 | -1,9 |
| gamma-interferon inducible protein 16 | -1,8 | -1,8 |
| poliovirus receptor | +2.0 | +2.5 |
| glypican 1 | +2,1 | +2.6 |

| | | |
|---|---|---|
| *⃝ ≙ comparative nucleic acid | | |

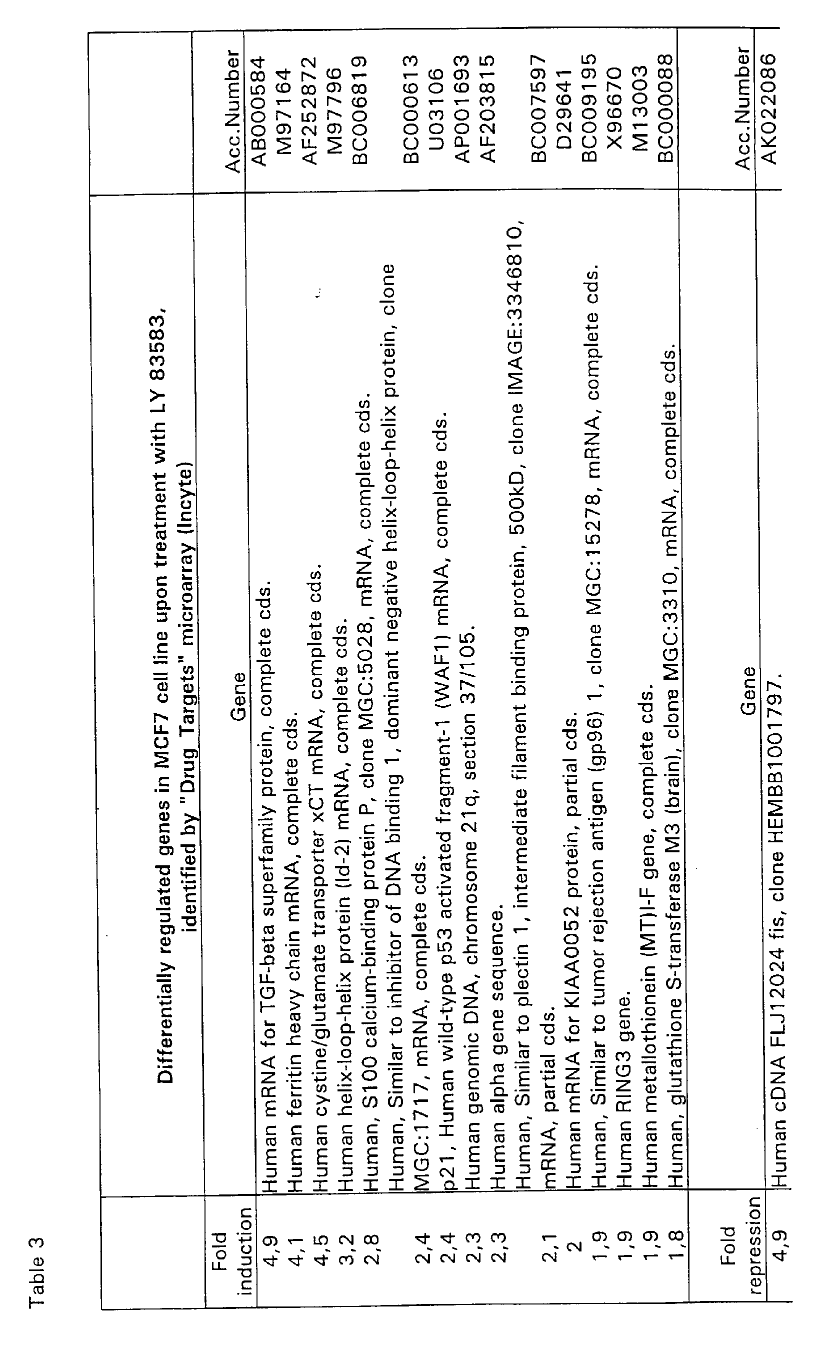

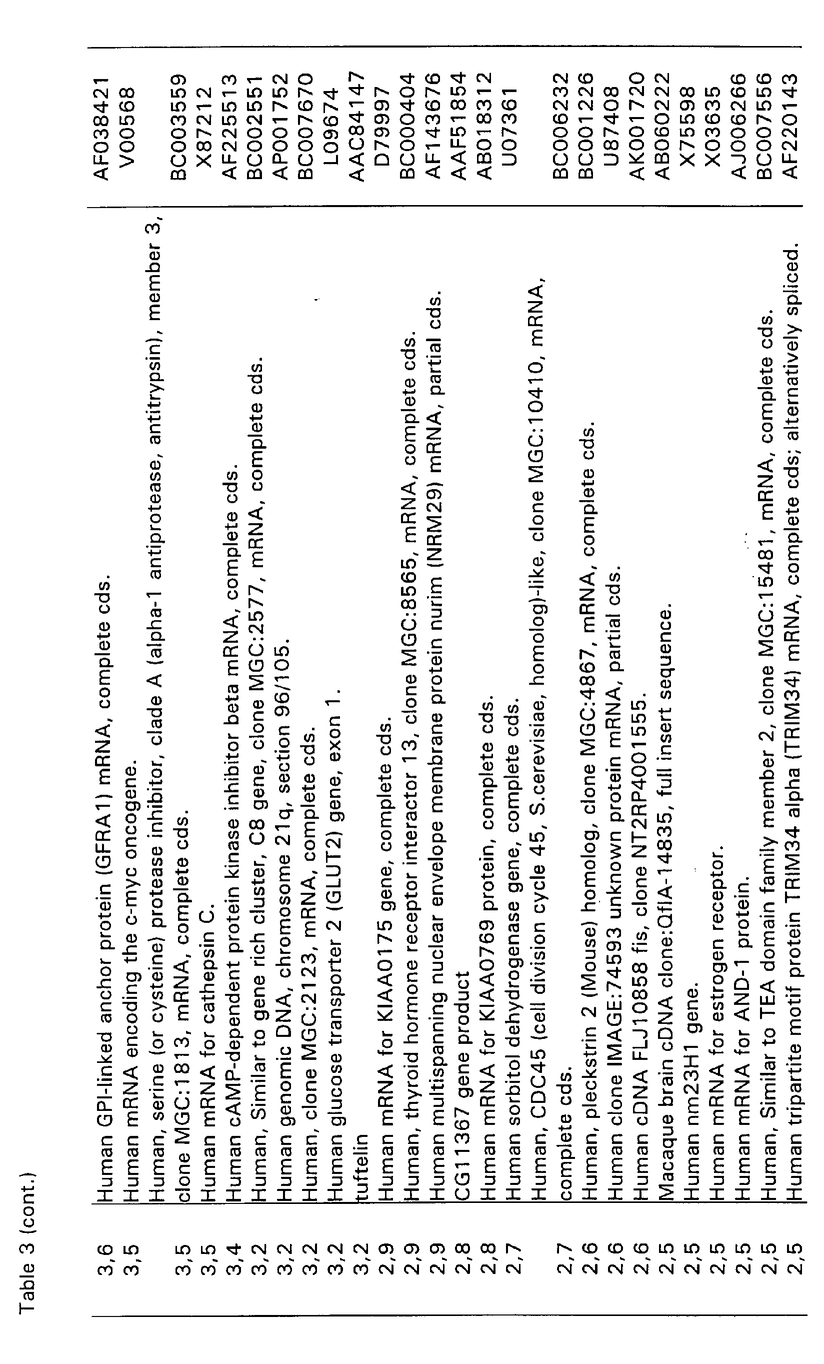

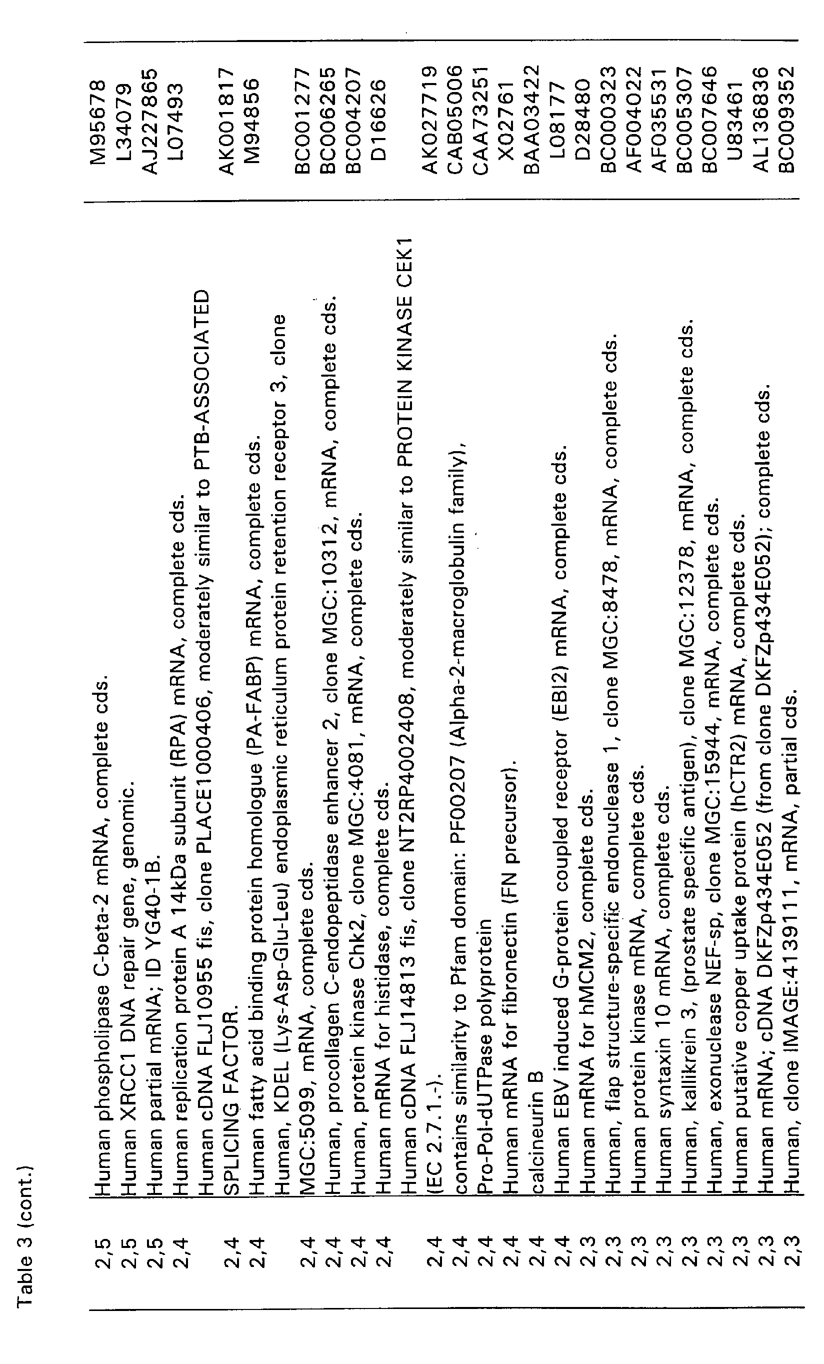

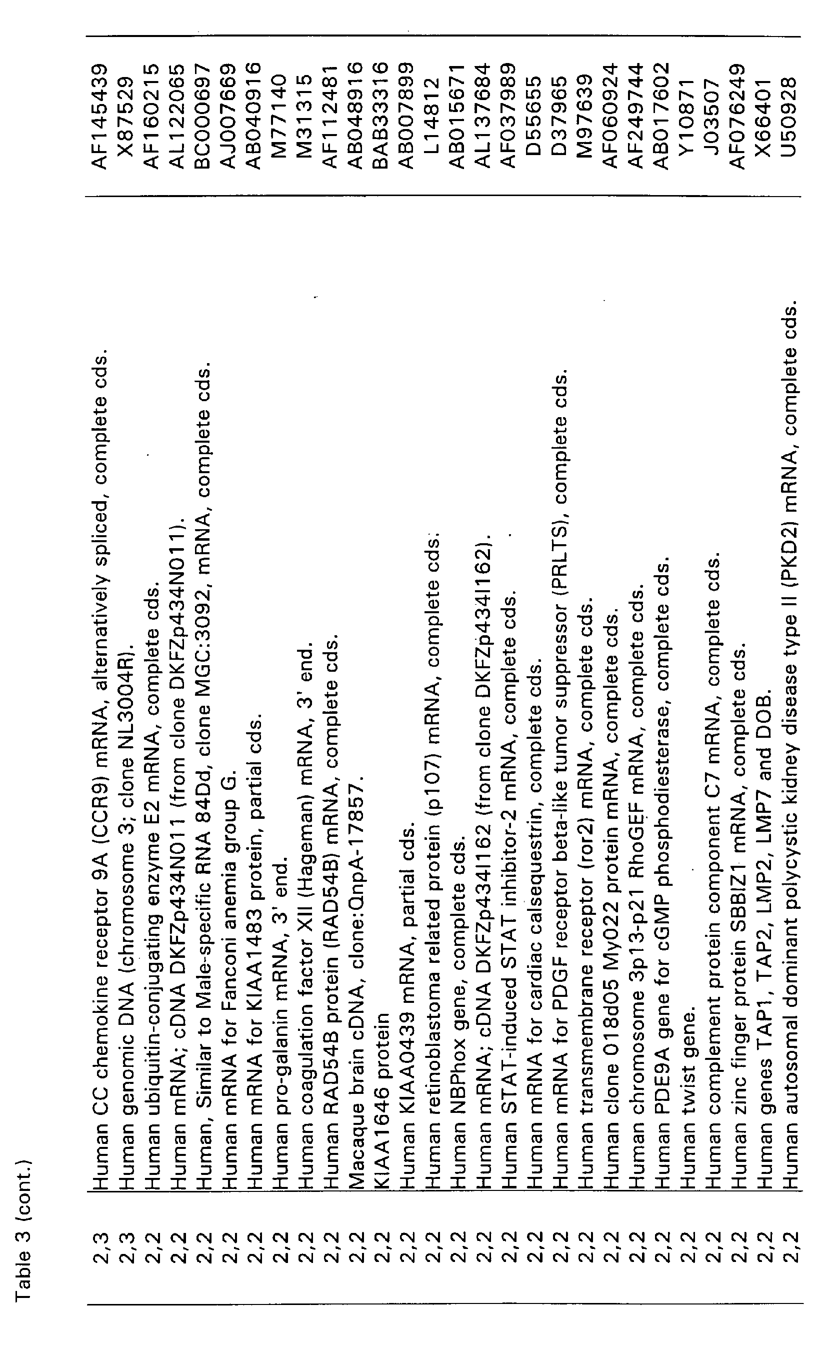

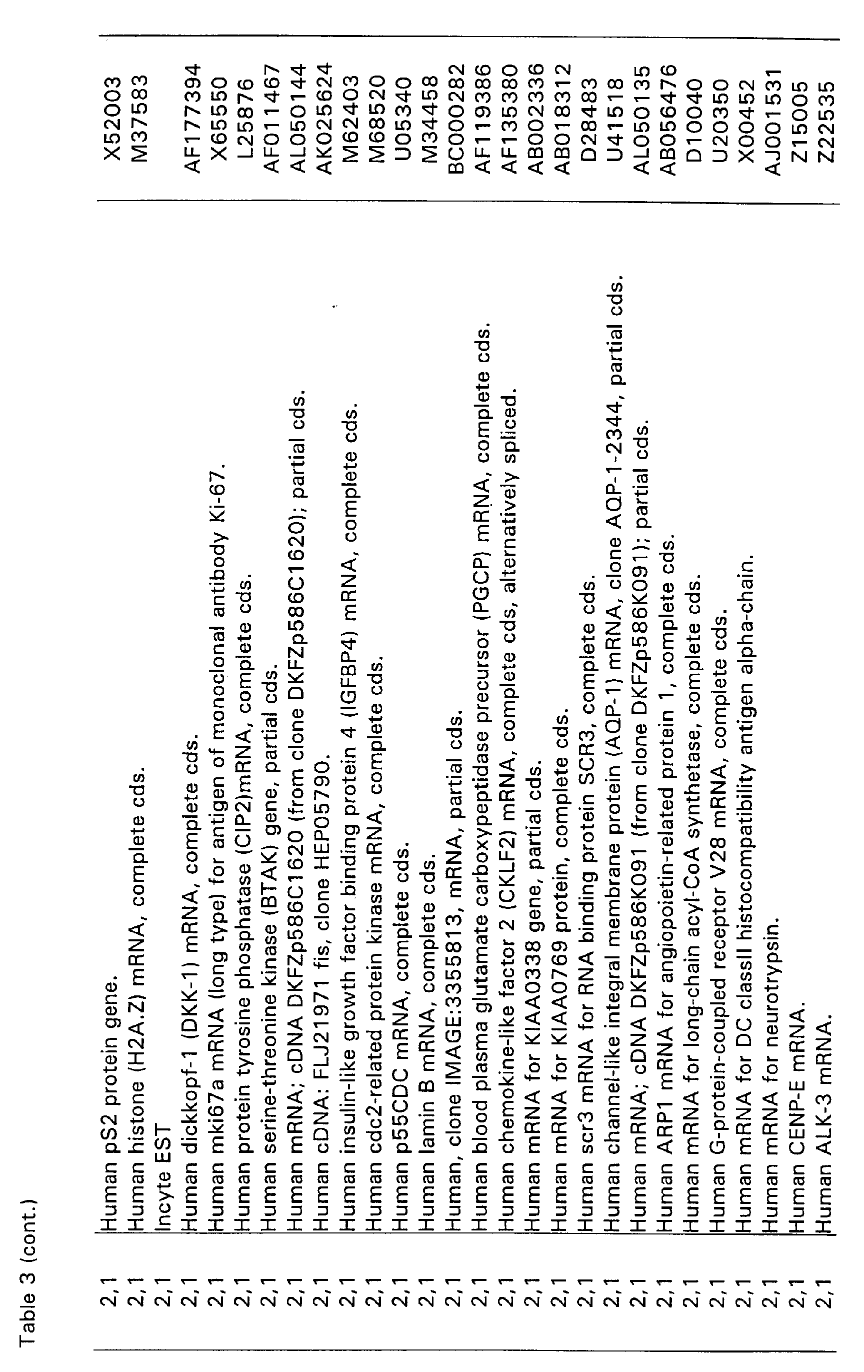

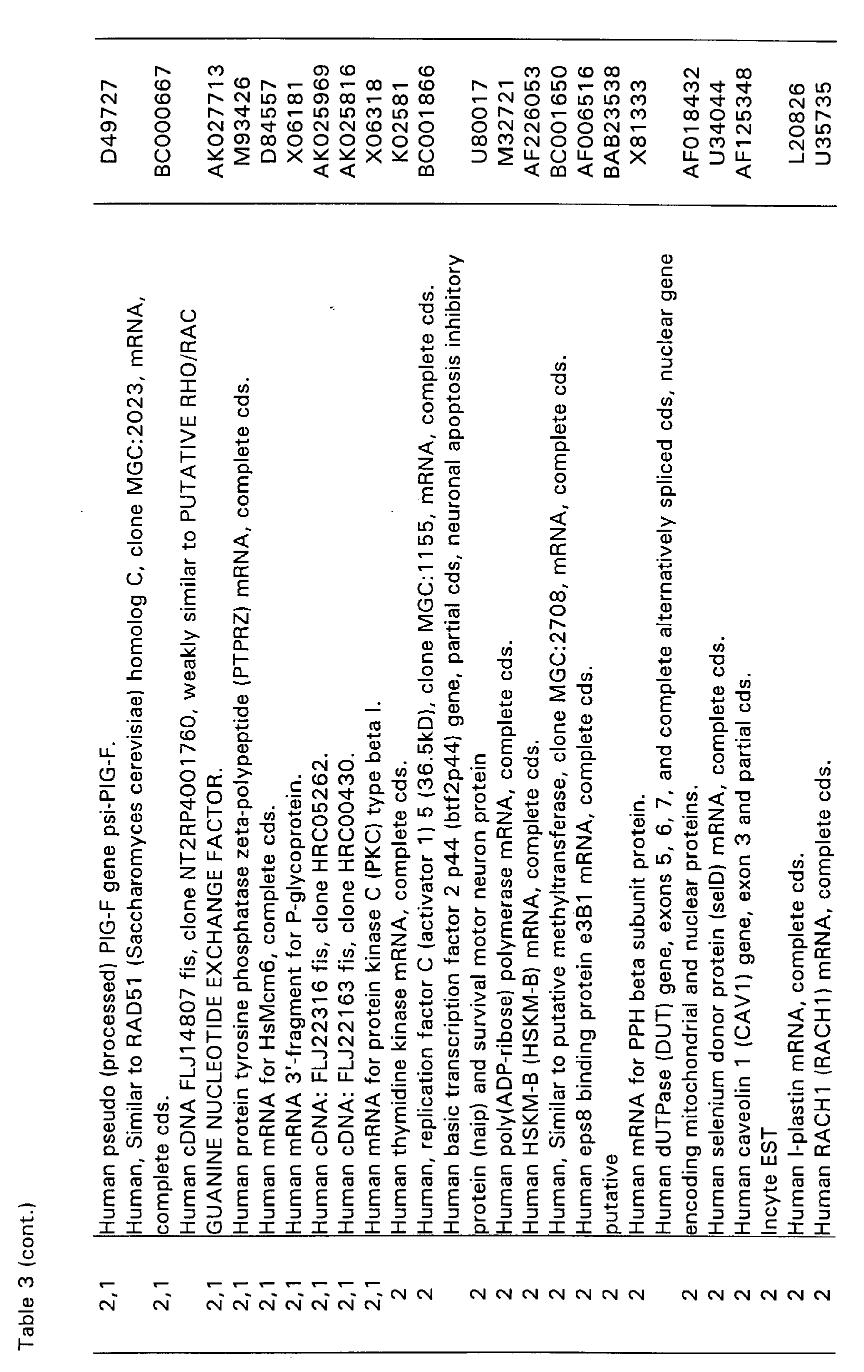

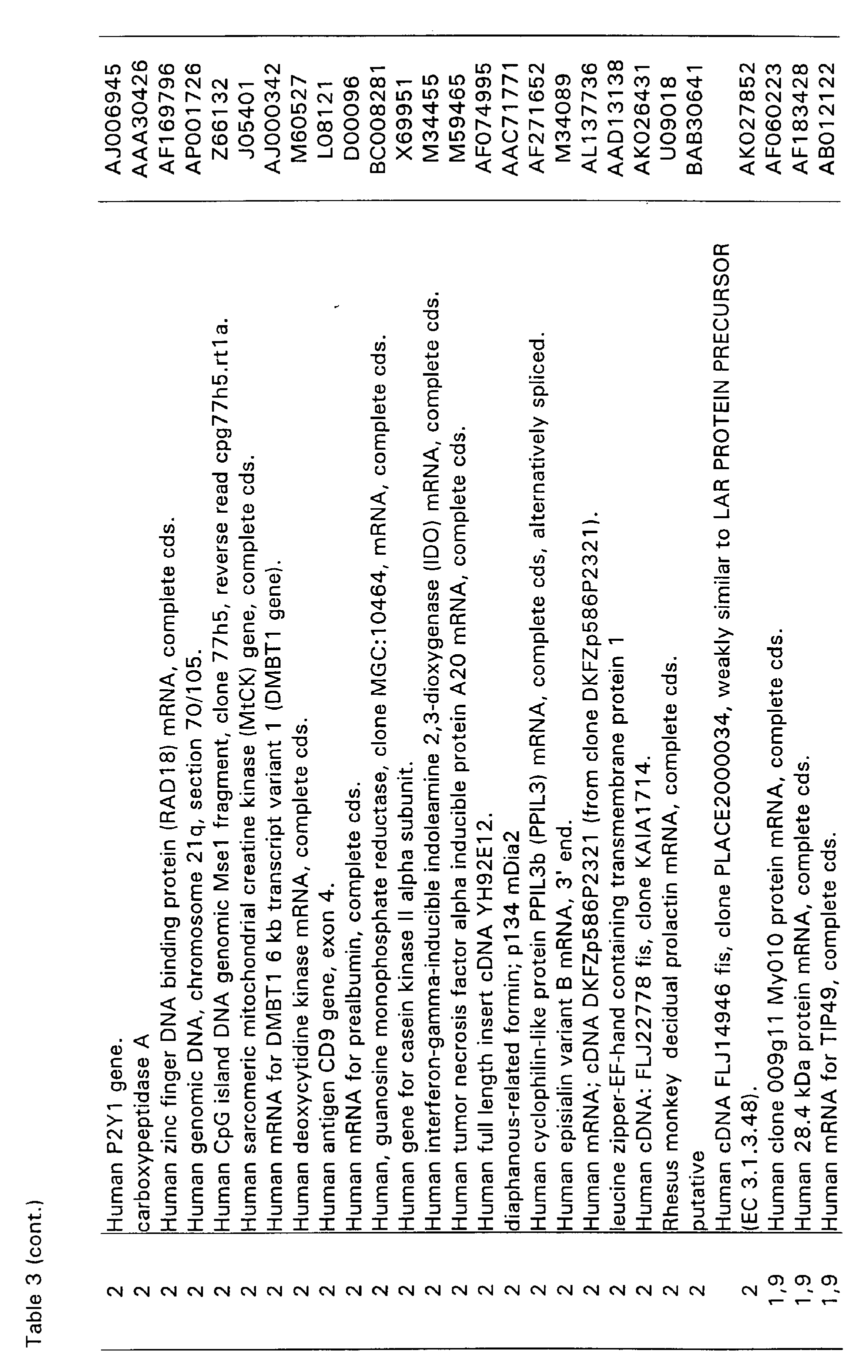

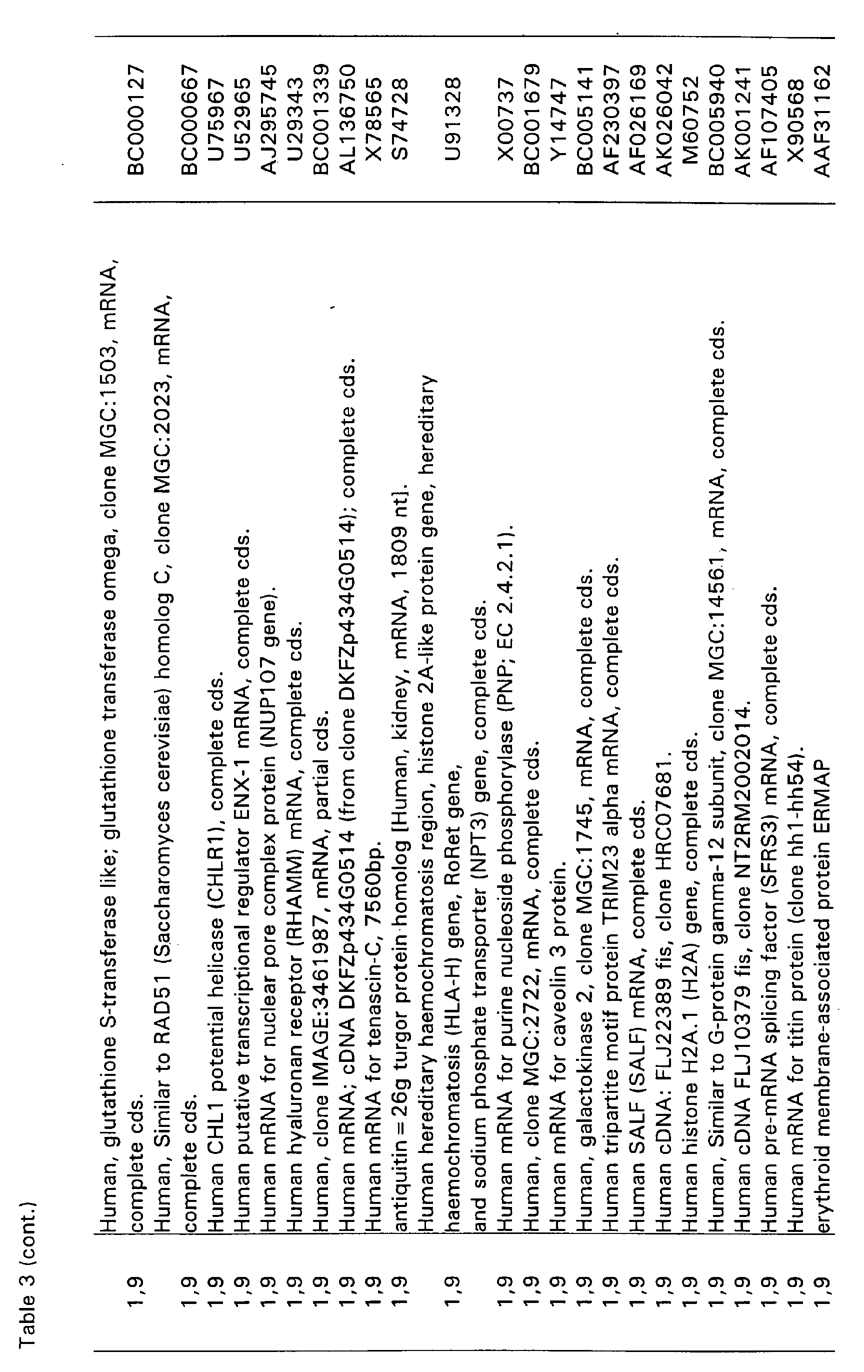

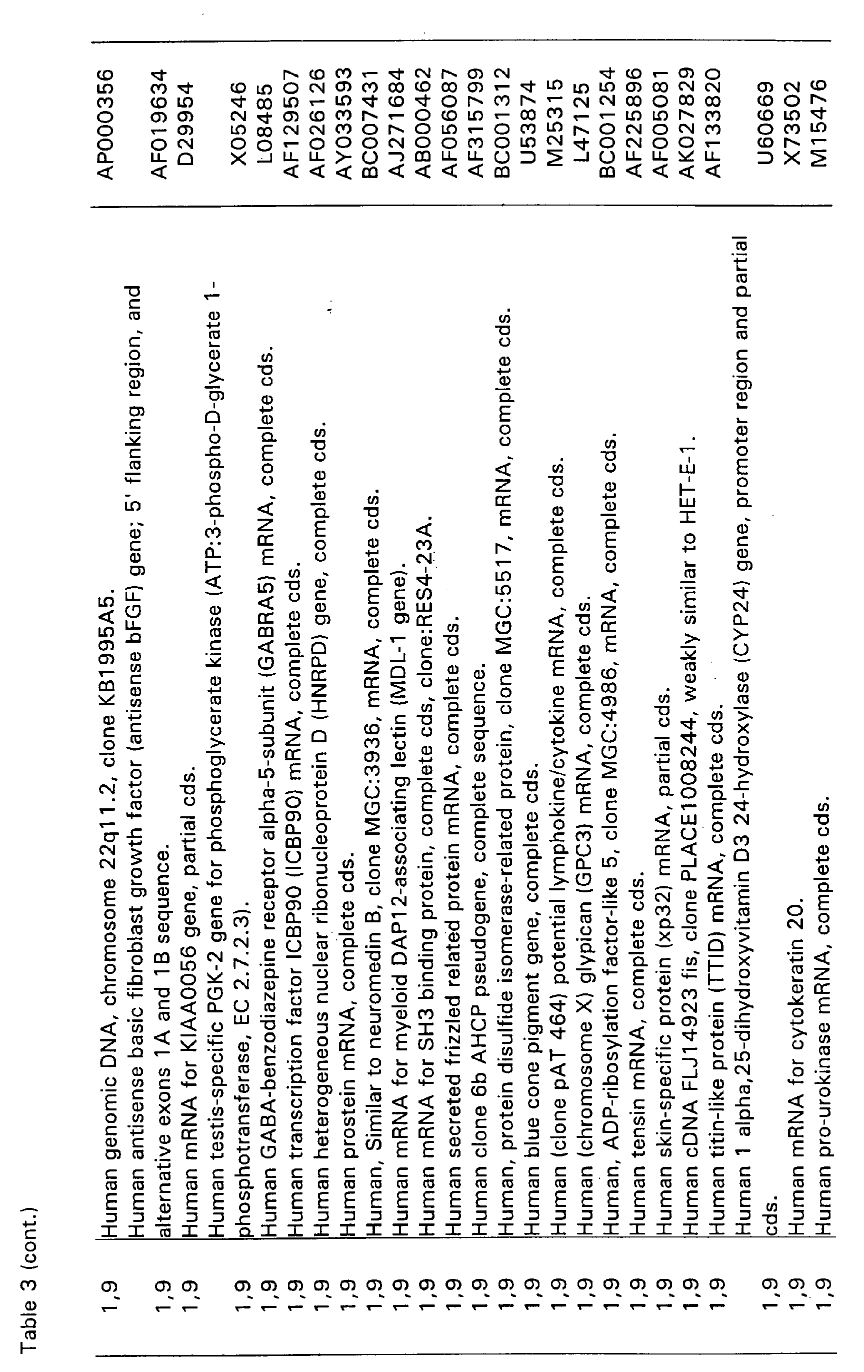

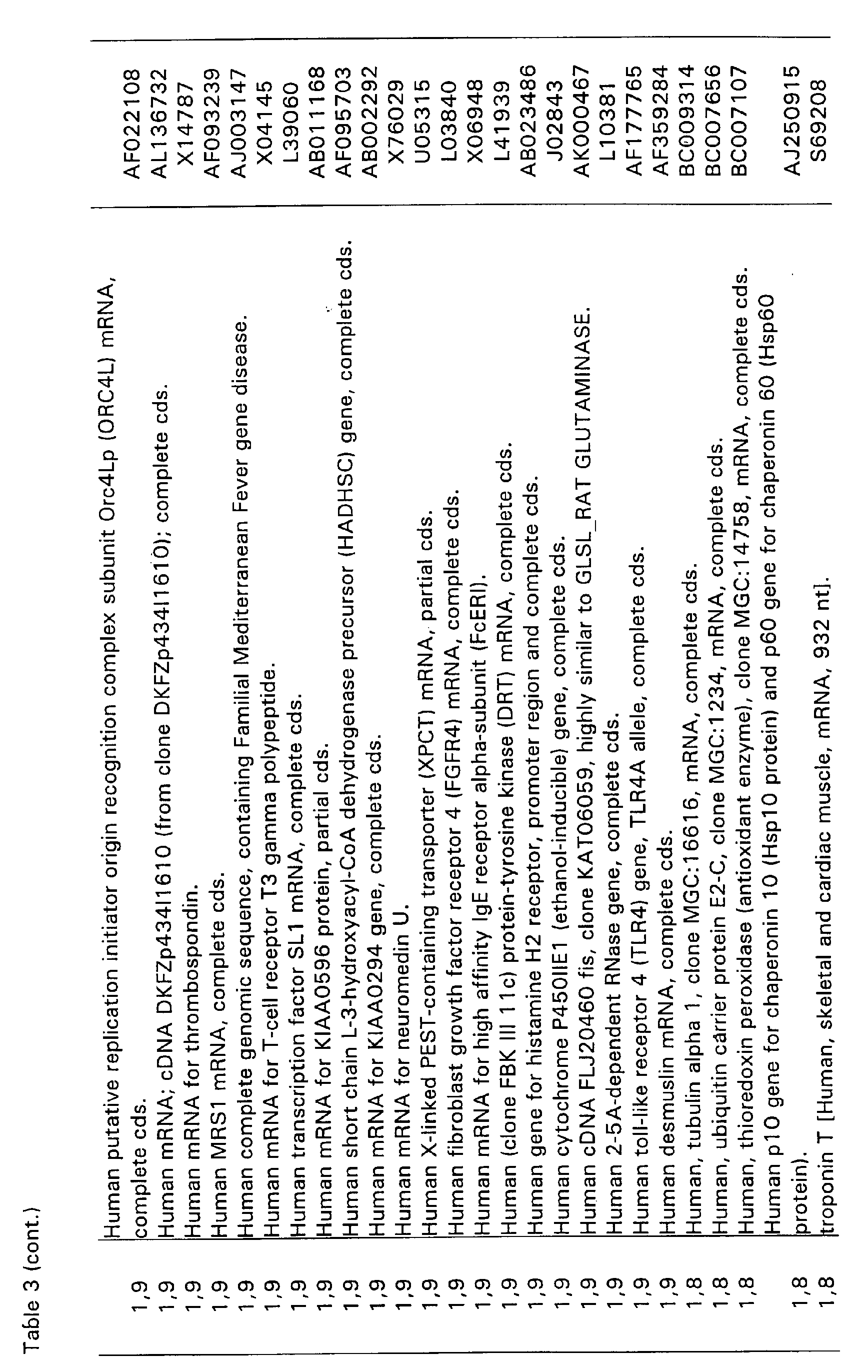

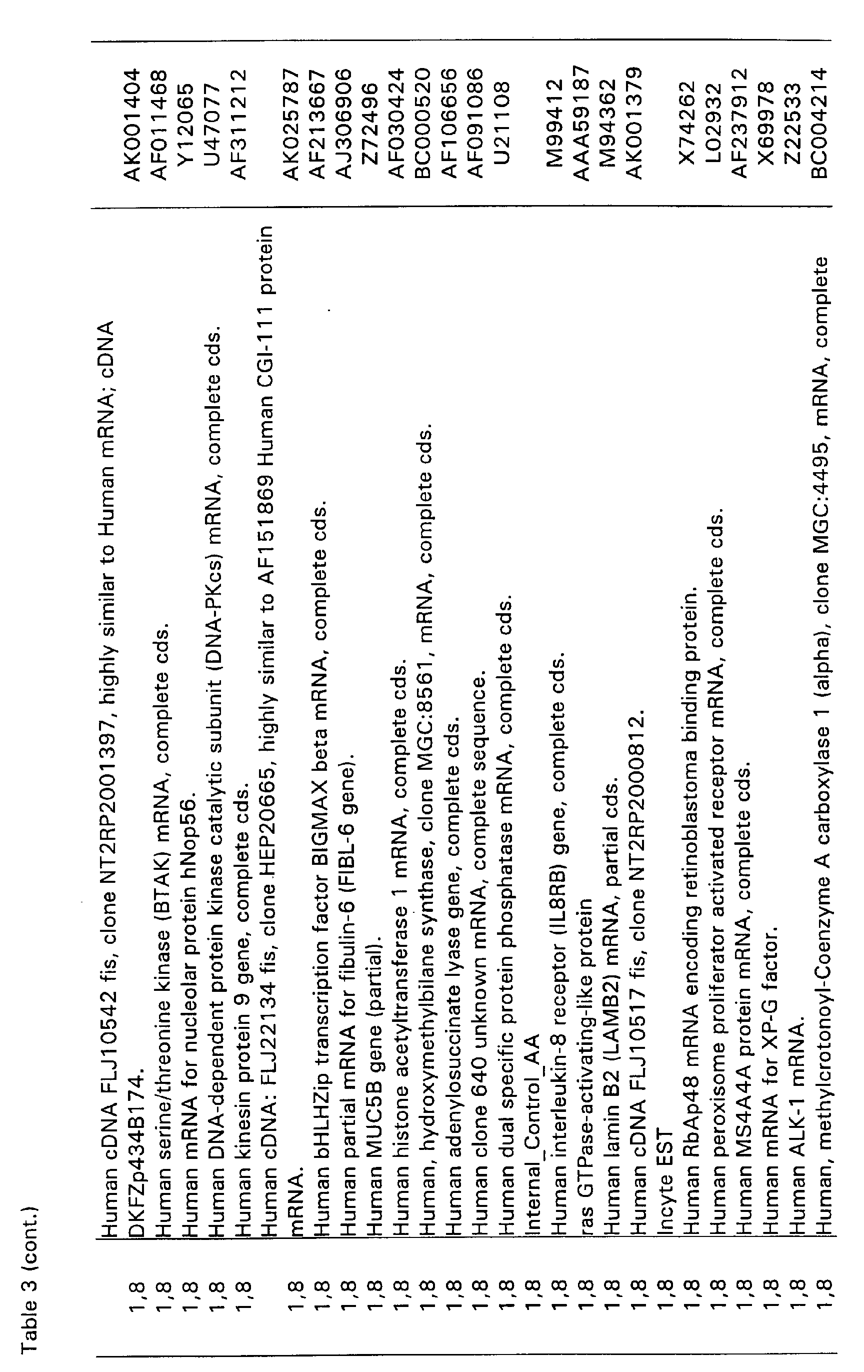

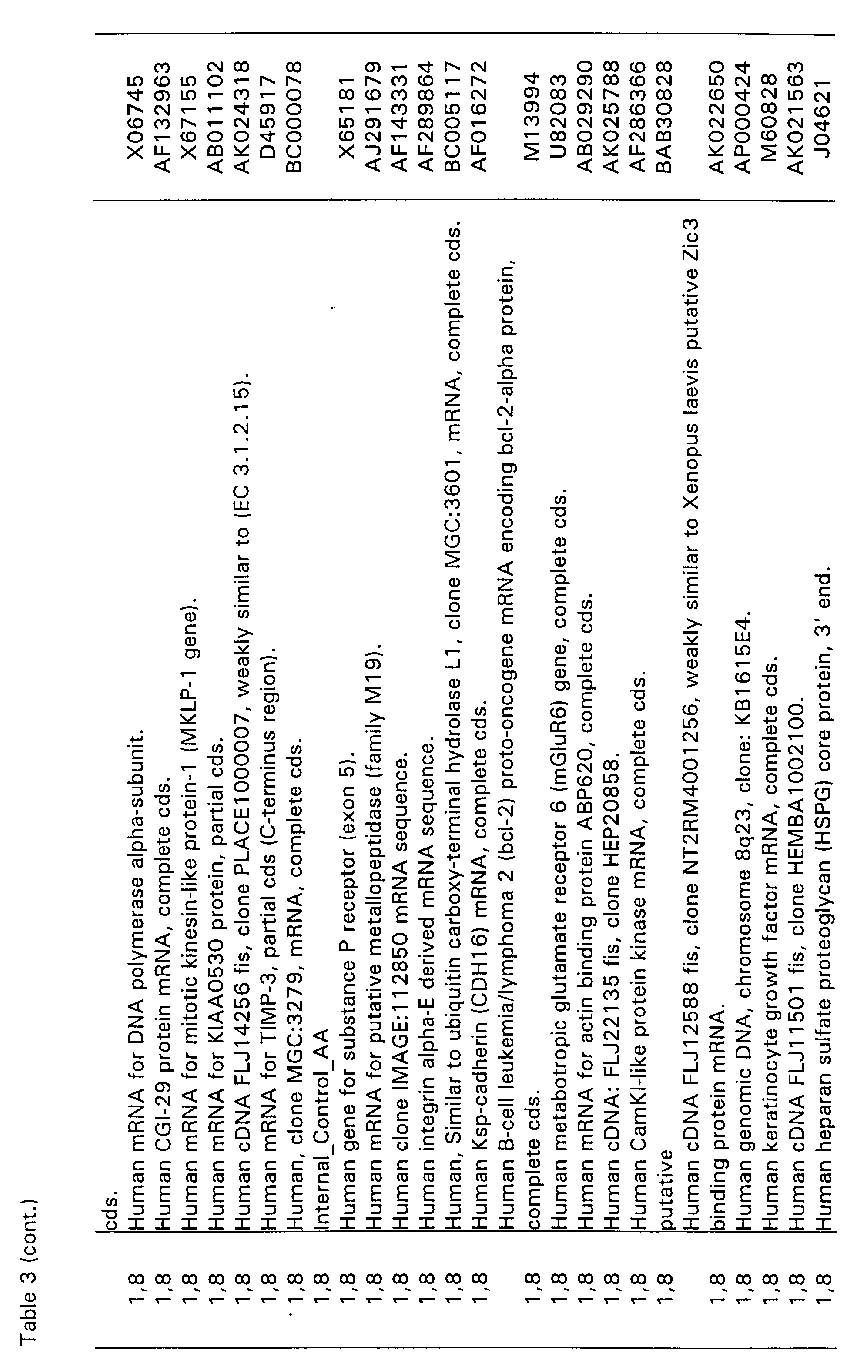

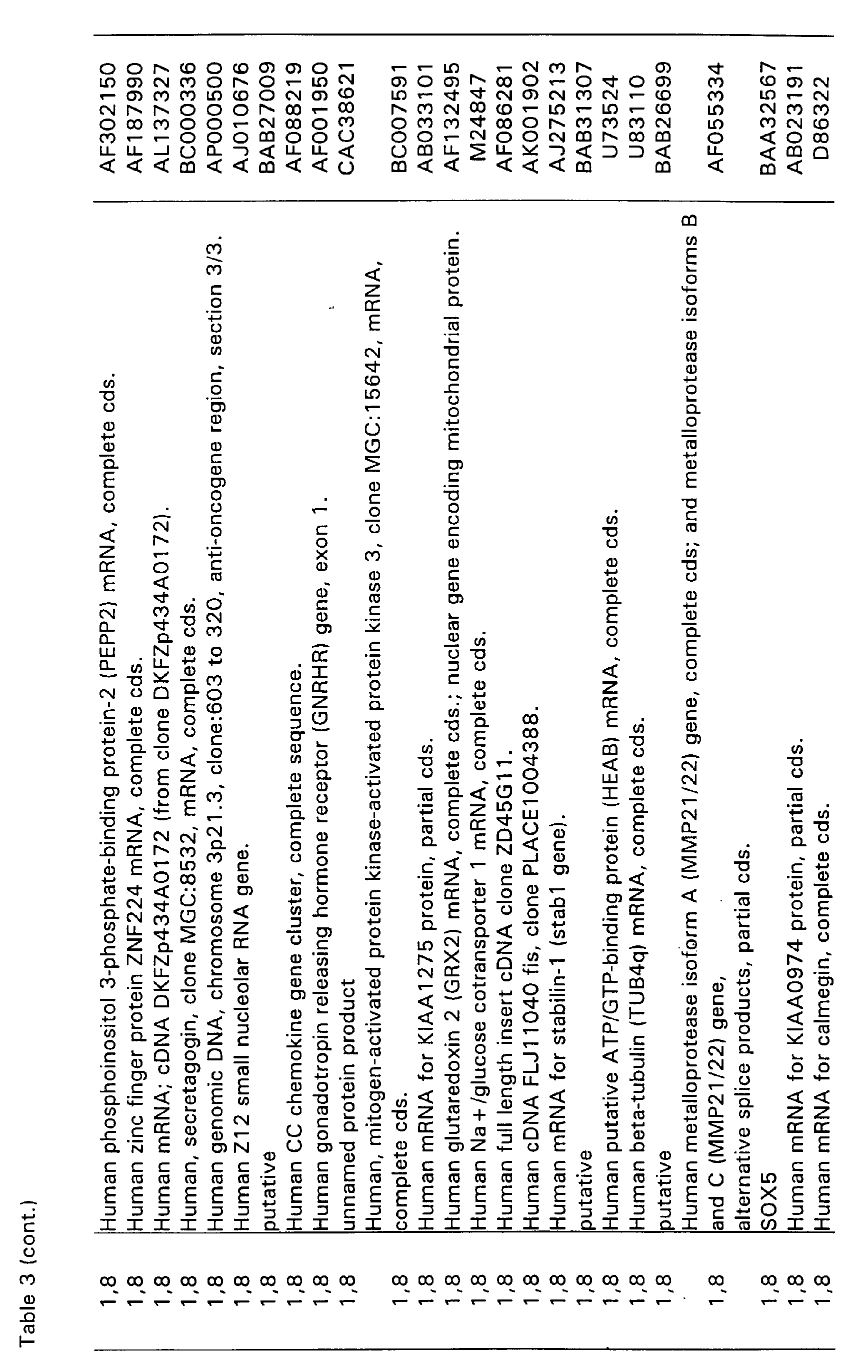

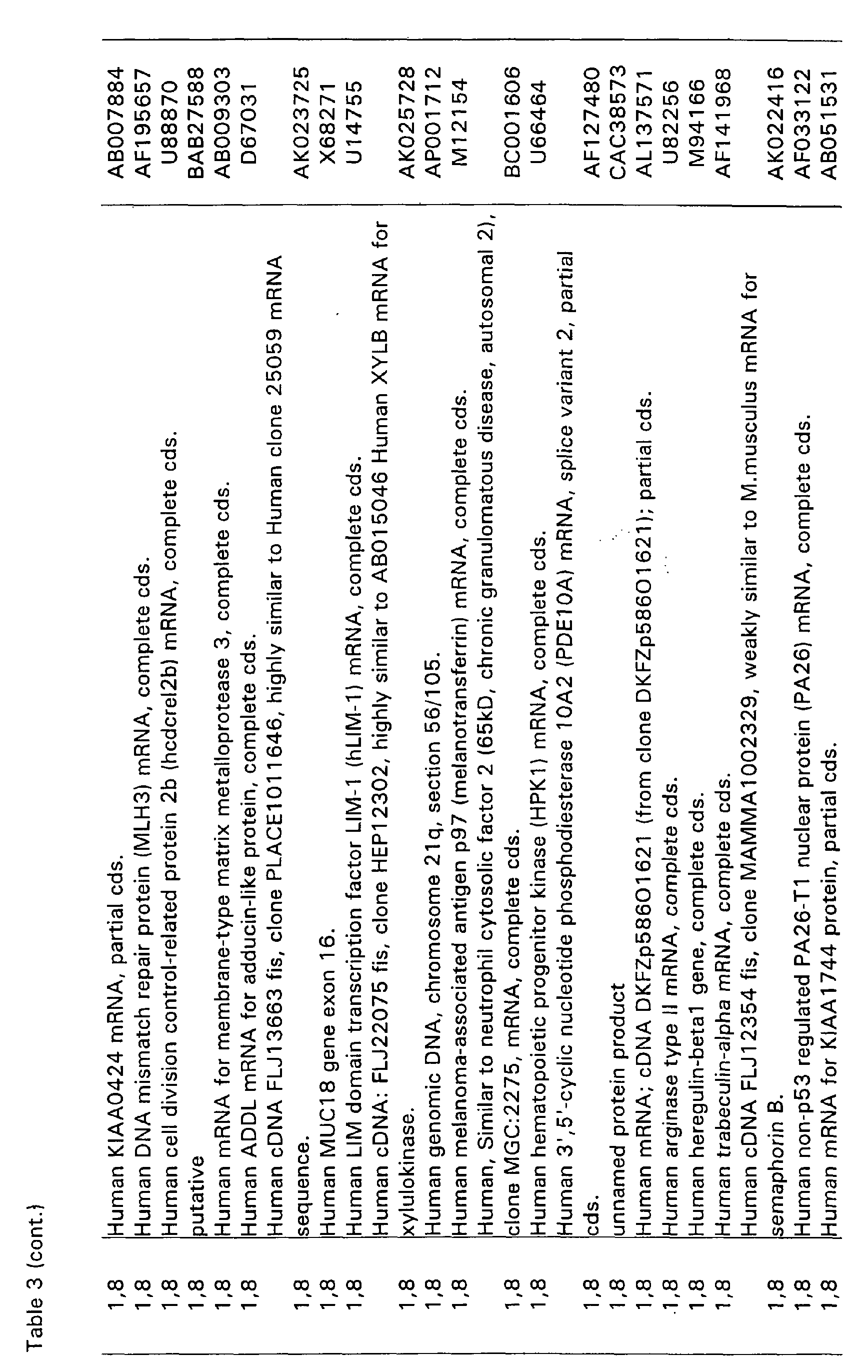

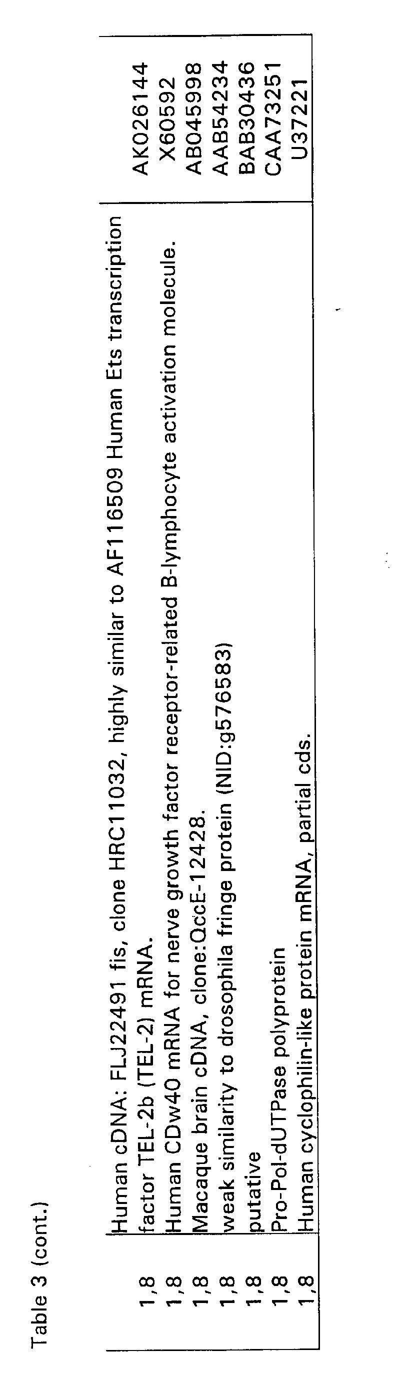

## Claims

1. Use of at least one 6-amino-quinoline-5,8-quinone for preparing a pharmaceutical composition for the treatment of tumors.

2. Use according to claim 1,
**characterized in that**
the 6-amino-quinoline-5,8-quinone is 6-anilino-quinoline-5,8-quinone.

3. Use according to claim 1 or 2,
**characterized in that**
the tumors are selected from intestinal cancer, brain tumor, eye tumor, pancreatic tumor, urinary bladder carcinoma, lung cancer, breast cancer, ovarian tumor, uterine carcinoma, bone tumor, gall bladder and bile duct carcinoma, head-neck tumor, urethral cancer, skin cancer, testicular cancer, renal tumor, germinal tumor, liver carcinoma, leucemia, gastric carcinoma, malignant lymphoma, adrenal carcinoma, neuroma, neuroblastoma, malignant melanoma, oesophageal cancer, prostate carcinoma, soft-tissue tumor, thymoma and carcinoma in the case of unknown primary cancer.

4. Use according to any one of claims 1 to 3,
**characterized in that**
the pharmaceutical composition is administered in a therapeutically effective amount to a patient suffering from a tumor disease.

5. Use according to claim 4,
**characterized in that**
the therapeutically effective amount is from 1 µg/kg of body weight of the patient to 5 mg/kg of body weight of the patient.

6. Use according to claim 5,
**characterized in that**
the pharmaceutically effective amount is from 10 µg/kg of body weight of the patient to 0.1 mg/kg of body weight of the patient.

7. Use according to any one of claims 4 to 6,
**characterized in that**
the pharmaceutical composition leads to inhibition of tumor cell proliferation by 6-amino-quinoline-5,8-quinone-induced activation of cellular senescence, cell cycle arrest and/or apoptosis.

8. Use according to any one of claims 1 to 7,
**characterized in that**
inhibition or reduction of tumor growth, reduction of tumor mass or inhibition or reduction of metastases can be observed.

9. Nucleic acid associated with senescence,
**characterized in that**
it encodes one of the proteins shown in Table 2.

10. Nucleic acid according to claim 9,
**characterized in that**
it induces senescence after being expressed in a cell.

11. Nucleic acid according to claim 9 or 10,
**characterized in that**
it is derived from a eukaryotic organism.

12. Nucleic acid according to claim 11,
**characterized in that**
it is derived from a mammal.

13. Nucleic acid according to any one of claims 9 to 12,
**characterized in that**
it is operatively linked with an expression control sequence.

14. Nucleic acid according to claim 13,
**characterized in that**
the expression control sequence is a heterologous expression control sequence.

15. Recombinant vector,
**characterized in that**
it contains a nucleic acid according to any of claims 9 to 14.

16. Recombinant cell,
**characterized in that**
it is transformed or transfected with a nucleic acid according to any one of claims 9 to 14 or a vector according to claim 15.

17. Polypeptide associated with senescence,
**characterized in that**
it is encoded by a nucleic acid according to any one of claims 9 to 14.

18. Pharmaceutical composition comprising a nucleic acid according to any one of claims 9 to 14, a vector according to claim 15 or a polypeptide according to claim 17, optionally together with conventional carrier substances and adjuvants.

19. Use of a nucleic acid according to any one of claims 9 to 14, a vector according to claim 15 or a polypeptide according to claim 17 for the preparation of a pharmaceutical composition for the treatment of diseases associated with hyperproliferation of cells.

20. Use according to claim 19 for the treatment of tumors.


SEQUENCE LISTING

<110> Max-Planck-Gesellschaft zur Förderung der Wissensc

<120> Use of 6-amino-chinoline-5,8-chinone and nucleic acids
      associated with senescence for the treatment of tumors

<130> 26342PEP_DR

<140>
<141>

<160> 4

<170> PatentIn Ver. 2.1

<210> 1
<211> 18
<212> DNA
<213> Artificial Sequence

<220>
<223> Description of Artificial Sequence: Primer

<400> 1
ttcagaggag gcagcagg                                                18

<210> 2
<211> 19
<212> DNA
<213> Artificial Sequence

<220>
<223> Description of Artificial Sequence: Primer

<400> 2
gcaacattca gccgttcaa                                               19

<210> 3
<211> 18
<212> DNA
<213> Artificial Sequence

<220>
<223> Description of Artificial Sequence: Primer

<400> 3
aggaatcacg catcagcc                                                    18

<210> 4
<211> 19
<212> DNA
<213> Artificial Sequence

<220>
<223> Description of Artificial Sequence: Primer

<400> 4
tatgaggacg aaccagcga                                                   19